# EUROPEAN PATENT APPLICATION

(11) **EP 4 613 860 A1**
(43) Date of publication of application: **10.09.2025**
(21) Application number: 24162111.9
(22) Date of filing: 07.03.2024
(51) Int. Cl.: C12N 15/113, A61K 31/712, C07K 19/00, C07K 7/08

(54) **INHIBITORS OF MIR-574-5P FOR USE IN INTRA-ARTICULAR TREATMENT OF OSTEOARTHRITIS AND OTHER RELATED ARTHRITIC DISEASES**

(71) Applicant: Technische Universität Darmstadt, 64289 Darmstadt (DE)
(72) Inventor: Saul, Meike Julia, 64653 Lorsch (DE); Breitwieser, Kai, 22525 Hamburg (DE); Steinhilber, Dieter, 61389 Schmitten (DE)
(74) Representative: Fuchs Patentanwälte Partnerschaft mbB

(57) **Abstract**

The present disclosure relates to inhibitors of miR-574-5p (SEQ ID NO: 1) for use in intra-articular treatment of a disease selected from the group consisting of osteoarthritis (OA), rheumatoid arthritis (RA), psoriatic arthritis, ankylosing spondylitis, gout, juvenile arthritis, systemic lupus erythematosus (SLE), Sjögren's syndrome and osteoporosis. (Figure 3)

## Description

The present disclosure relates to an inhibitor of miR-574-5p (SEQ ID NO: 1) for use in intra-articular treatment of a disease selected from the group consisting of osteoarthritis (OA), rheumatoid arthritis (RA), psoriatic arthritis, ankylosing spondylitis, gout, juvenile arthritis, systemic lupus erythematosus (SLE), Sjögren's syndrome and osteoporosis.

### BACKGROUND

OA is the most common chronic joint disease, affecting an estimated 340 million adults worldwide. It is a serious disease that significantly affects patients' quality of life. The disease is characterized by inflamed joints with pain and progressive joint destruction and loss of function. OA is caused by daily wear and tear of the joints (primary OA) or by traumatic injury (post-traumatic OA). The incidence of OA increases with age. In Europe, OA affects almost half of women and one third of men over the age of 65. The incidence and prevalence of OA will continue to increase due to an aging population, rising obesity rates, and high rates of traumatic knee injury.

Any joint can be affected by OA. The joints that are most commonly affected are the knees, hips, hands, and toes. A feeling of stiffness and tightness in the affected joint is often the first sign of OA. As the disease progresses, pain develops on exertion, and later permanent pain with restriction of movement. Curative treatment is not available. In contrast to OA, rheumatoid arthritis is relatively amenable to treatment. Drugs such as methotrexate interfere with the immune system and delay or suppress joint destruction. All OA treatments aim to relieve pain and re-store range of motion. Pain is most commonly treated with non-steroidal anti-inflammatory drugs such as aspirin. These prostaglandin (PG) inhibitors are taken symptomatically for their anti-inflammatory effects. However, they are associated with serious side effects. For example, bleeding, gastrointestinal and renal side effects may be observed with long-term therapy.

Surgical treatment strategies are sought in the final stages. For example, in elderly patients, artificial hip or knee joints are often implanted. However, these approaches provide a number of disadvantages and risks, such as infection, blood clots, implant wear and tear, implant loosening, fractures, nerve and blood vessel damage, pain and discomfort, limited range of motion, allergic reactions, scar tissue formation, rehabilitation challenges and costs.

Therefore, inhibition of bone resorption and associated joint pain is an unmet medical need in OA.

### DESCRIPTION OF THE INVENTION

In a first aspect the disclosure relates to an inhibitor of miR-574-5p (miR-574-5p having SEQ ID NO: 1) for use in intra-articular treatment of a disease selected from the group consisting of osteoarthritis (OA), rheumatoid arthritis (RA), psoriatic arthritis, ankylosing spondylitis, gout, juvenile arthritis, systemic lupus erythematosus (SLE), Sjögren's syndrome and osteoporosis.

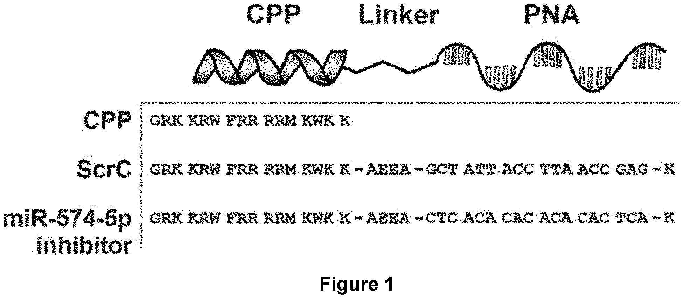

In one embodiment the inhibitor is disclosed as "miR-574-5p inhibitor" in figure 1 and comprises the sequences according to SEQ ID NO: 6 and SEQ ID NO: 13.

In second aspect the disclosure relates to a pharmaceutical composition comprising the inhibitor as disclosed hereinunder.

Bone resorption is mediated by osteoclasts and plays an important role in various diseases. In particular, a negative influence of bone resorption has been described in connection with joint diseases. This concerns acute joint diseases such as arthritis, but also chronic joint diseases such as rheumatoid arthritis (RA) and arthrosis. Excessive bone resorption also plays an important role in other diseases such as periodontitis or implants that grow poorly or not at all.

Chronic joint diseases (arthropathies) are the most common chronic diseases of older individuals in Germany. The pathology of arthropathies is complex and multifactorial, but the pathogenesis generally involves inflammation of the joints accompanied by bone resorption, leading to chronic pain and physical impairment. Two of the most common types of arthropathies are rheumatoid arthritis and arthrosis.

Osteoarthritis (OA) is caused by daily wear and tear of the joints or by traumatic damage. Older people are therefore most often affected. Half of all women and one third of all men develop arthrosis after the age of 60. Currently, there is no curative treatment for any of these diseases. Today's therapies are aimed solely at relieving pain and maintaining the patient's mobility.

The present invention is therefore based on the task of providing substances for the curative treatment of osteoarthritis. The task is solved by the subject-matter of the claims.

It was discovered that increased microRNA (miRNA) miR-574-5p expression is directly associated with increased prostaglandin E2 (PGE2) synthesis. PGE2 is an important bioactive lipid mediator involved in inflammation and pain. In addition, it was demonstrated that miR-574-5p acts extracellularly as a ligand for toll-like receptor (TLR) 7/8. Thus, miR-574-5p acts as a hormone-like signalling agent to induce bone resorption by enhancing osteoclast maturation. The central role of miR-574-5p in inflammation and osteoclast-mediated bone resorption makes this miRNA a promising drug target for the treatment of OA. An inhibitor of miR-574-5p targets both its intracellular function as a regulator of prostaglandin synthesis and its extracellular function as a facilitator of osteoclast genesis.

Disclosed is the use of ribonucleic acid (RNA)-based therapeutics to inhibit miRNA functions and to modulate the expression of disease-relevant genes. Thus, an RNA-based drug can intervene in the disease process more upstream than a conventional drug. Disclosed is also a PNA-based miR-574-5p antagonist which is coupled to a cell-penetrating peptide to allow cell entry and antagonism of miR-574-5p.

The current treatment of inflammation and pain consists of oral and topical application of acetaminophen and non-steroidal anti-inflammatory drugs (NSAIDs). However, long-term use of NSAIDs can lead to serious side effects and recent meta-analyses of placebo-controlled knee OA trials question the long-term efficacy of NSAIDs and acetaminophen in providing effective pain relief. However, for patients with severe, late-stage OA, surgery and joint replacement remain the only viable treatment options. Therefore, there is an urgent need for the development of new disease-modifying osteoarthritis drugs (DMOADs) that can effectively halt or slow disease progression.

To achieve this, an antisense oligonucleotide (ASO) approach is disclosed. ASO therapies have the advantage of modifying the gene expression of their target cells. Hence, preventing the expression of pathophysiological relevant genes instead of inhibiting their function, as for example competitive small molecule inhibitors. The magnitude of this effect can be further amplified by targeting miRNAs, which can influence gene expression of multiple targets. Thus, inhibition of a single miRNA by an ASO can affect a variety of physiological processes simultaneously. Although proper candidate selection requires comprehensive knowledge of the miRNA and its downstream effects, miRNA-ASO-therapy may offer greater therapeutic effects than conventional ASO-therapy.

The disclosure relates to miR-574-5p as a candidate therapeutic target for OA. It is disclosed that high intracellular miR-574-5p levels are associated with increased synthesis of prostaglandin E2, a key mediator of inflammation and pain. Moreover, it is disclosed that miR-574-5p induces osteoclast differentiation via direct binding to Toll-like receptors 7/8. This newly discovered link between miR-574-5p, inflammation and osteoclast-mediated bone resorption provided an opportunity to develop novel RNA therapeutics for OA. Disclosed is a first-in-class miR-574-5p inhibitor that simultaneously suppress inflammatory pain, bone resorption and cartilage degeneration without toxic side effects. The inhibitor is based on a peptide nucleic acid (PNA) sequence conjugated to a cell-penetrating peptide (CPP). The drug is designed for intra-articular administration to minimize potential non-specific side effects, such as infections, tissue damages, bruises, swellings, allergic reactions and to provide high local concentrations. The results of the in-vivo study in the anterior cruciate ligament transection (ACLT) mouse model confirm that the inhibitor halts the progression of OA, restores joint functionality and, at the same time, significantly reduces inflammation.

The present invention relates to a miR-574-5p inhibitor for use in reducing bone resorption, particularly in chronic joint disease, and a pharmaceutical composition comprising the inhibitor.

miR-574-5p belongs to the class of so-called microRNAs (miRs), a class of small, non-coding RNAs with a function in gene regulation. Extracellular miRs are secreted in so-called small extracellular vesicles (sEVs) in human body fluids such as synovial fluid, so that they are protected from degradation by ribonucleases.

Functionally, two fundamentally different main modes of action of miRs can be distinguished. On the one hand, miRs bind to their target mRNA in a sequence-dependent manner and provide for their translational repression or degradation. On the other hand, miRs can also activate gene expression by binding to RNA-binding proteins and inhibiting their functions. Also, miR-574-5p has been identified as a TLR7/8 ligand. However, a role of miRs in osteoarthritis (OA) was not known until now. In particular, it was also not known that EV-mediated miRs can act similarly to a hormone.

It was found that miR-574-5p plays a decisive role in connection with bone resorption, especially in chronic joint diseases, such as osteoarthritis (OA).

The present invention thus relates to a miR-574-5p inhibitor for use in the treatment of osteoarthritis. In one embodiment the inhibitor is a miR-574-5p inhibitor comprising or consisting of peptidic nucleic acid (PNA). In one embodiment it may be a PNA-based antagomiR against miR-574-5p.

Various ways of inhibiting miRs are known to the skilled person and are described, for example, in US 2017/0044541 A1 or US 2009/0286852 A1. Various antisense methods (e.g., siRNA) are also known to the person skilled in the art. Providing a suitable inhibitor against a given miR is therefore readily possible.

In one embodiment the inhibitor may be a single-stranded RNA complementary to miR-574-5p (SEQ ID NO: 1) or a single-stranded RNA-analogon (antagomiR) complementary to miR-574-5p. In another embodiment the inhibitor consists of and/or comprises SEQ ID NO: 2, 3, 4, 5, or 42, (in case of RNA, the nucleotide sequence may comprise uracil instead of thymine). If the inhibitor consists of nucleic acid, optionally only of nucleic acid sequences according to SEQ ID NO: 2, 3, 4, 5, or 42, it may also be called "nucleic acid-based inhibitor".

Thus, in one embodiment the sequence of the nucleic acid-based inhibitor consists of a nucleic acid sequence selected from the group consisting of SEQ ID NO: 2, 3, 4, 5, and 42, as well as truncated versions thereof. In another embodiment the sequence of the nucleic acid-based inhibitor is at least 90%, at least 95%, at least 99% complementary to miR-574-5p (SEQ ID NO: 1) and, optionally, the nucleic acid-based inhibitor is single-stranded.

In one embodiment the nucleic acid may be selected from the group consisting of LNA ("locked nucleic acid"), BNA ("bridged nucleic acid"), PMO ("phosphorodiamidate morpholino oligomer") and PNA ("peptide nucleic acid"), wherein the RNA or the RNA-analogon is completely complementary to miR-574-5p but does not cover the entire sequence of miR-574-5p. In one embodiment the nucleic acid may be LNA, in another embodiment the nucleic acid may be PNA (the corresponding PNA sequence is shown in SEQ ID NOs: 4 and 5).

In one embodiment, the inhibitor is an antagomiR represented by SEQ ID NO: 2 and 3, or 42. In yet another embodiment the inhibitor is a miR-574-5p is a PNA antagomiR represented by SEQ ID NO: 4 or 5. In further embodiments the antagomiR is further coupled with a CPP according to SEQ IDs NO: 6 - 12, or 25 - 33, or 41 directly or indirectly via a linker, such as a PEG-linker or AEEA-linker. In one embodiment the inhibitor is depicted in figure 1. Compared to the sequences of SEQ ID NOs: 2 and 4 covering the entire sequence of miR-574-5p, the sequences of SEQ ID NOs: 3 and 5 are five residues shorter, but still may inhibit miR-574-5p (miR-574-5p being depicted in SEQ ID NO: 1). SEQ ID NO: 42 is even shorter than sequences 3 and 5, but still shows miR-574-5p-inhibition.

In yet another embodiment the inhibitor is not the miR-574-5p PNA antagomiR of SEQ ID NO: 4 or 5 and/or the antagomiR SEQ ID NO: 4 or 5 is not coupled with a CPP according to SEQ ID NO: 6-12, or 25-33, or 41 directly and/or indirectly via a PEG-linker.

Other miR-574-5p inhibitors are also disclosed. For example, the invention also includes so-called "miRNA sponge" and "miRNA decoy". These are nucleic acid molecules that contain tandem binding sites for miR-574-5p. Thus, they act as competitive inhibitors of miR-574-5p (Ebert and Sharp, 2010 and US 2017/0044541 A1).

In one embodiment the miR-574-5p inhibitor is selected from the group consisting of a antagomiR represented by SEQ ID NO: 2-5 as well as truncated and/or mutated variants therefrom, CPP-antagomiR-conjugate, miRNA sponge and miRNA decoy, as well as combinations thereof.

In a further embodiment the inhibitor of miR-574-5p comprises a nucleic acid at least 90%, at least 95%, at least 99% complementary to miR-574-5p (as represented by SEQ ID NO: 1) and, optionally, a single-stranded nucleic acid. In a further embodiment the inhibitor of miR-574-5p comprises a PNA at least 90%, at least 95%, at least 99% complementary to miR-574-5p (as represented by SEQ ID NO: 1), and, optionally a CPP.

As outlined above, in some embodiments of the present disclosure, the inhibitor may comprise a cell penetrating peptide (CPPs), in one embodiment the inhibitor may be a CPP-antagomiR-conjugate.

CPPs are polycationic peptides that bring a variety of benefits. In one embodiment the cell-penetrating peptide (CPP) may be selected from the group consisting of a CPP according to SEQ ID NO: 6, penetratin (SEQ ID NO: 7), transactivator of transcription (TAT) (SEQ ID NO: 8), model amphiphatic peptide (MAP) (SEQ ID NO: 9), polyarginines (including R3, R4, R5, R6, R7, R8, R9, R10, R11 and R12) (SEQ ID NO: 10 or SEQ ID NO: 25-33), pVEC (SEQ ID NO: 11), and transportan (SEQ ID NO: 12), as well as functional derivatives, peptidomimetics and combinations thereof.

"Functional derivatives" include CPPs that consist of or comprise the above-mentioned sequences, or sequences having at least 90%, or at least 95%, sequence identity therewith. Optionally, the functional derivative may include additional amino acids.

"Peptidomimetics" are molecules designed to mimic the structure and/or function of naturally occurring peptides.

In an embodiment the CPPs used in this disclosure are positively charged and/or cyclized. Cyclized CPPs have the advantage of being less reactive and more stable than linear CPPs which is advantageous within the concept of this disclosure. As used herein, the term "cyclized" is not to be construed as relating to a peptide having one ring system only, i.e., the present invention is not limited to monocyclic peptides. Accordingly, the present disclosure includes cyclopeptides wherein two or more ring systems are covalently linked to each other. Furthermore, the cyclopeptides may also comprise amino acids which are not part of the ring system, i.e., the disclosure includes branched cyclopeptides. In one embodiment the cyclopeptides are monocyclic peptides and may be unbranched monocyclic peptides. Further, the CPPs can be composed of L-amino acids, D-amino acids, or mixtures thereof, wherein for linear CPPs, in one embodiment D-amino acids.

In an embodiment, the CPPs comprise a majority of lysine and/or arginine moieties, which have isoelectric points of around 9.5 and 11, respectively. Due to their additional amino or guanidine group, these two amino acids are positively charged under neutral and even under weakly basic conditions. Accordingly, a CPP mostly comprising moieties of said two specific amino acids is positively charged under neutral and weakly basic conditions as well. Herein, the term "majority" means that at least 50%, at least 60%, at least 70%, at least 80% of the amino acids forming the CPP molecule are lysine and/or arginine moieties. Thereby, it is ensured that the CPPs have a positive charge under neutral and weakly basic conditions, i.e., have an isoelectric point of more than 7. Therefore, in a specific embodiment of the present invention, the CPPs have an isoelectric point of more than 7.0, of more than 7.5, of more than 8.0, of more than 8.5. In one embodiment of the present invention, the CPPs have an isoelectric point from 7.0 to 9.5, from 7.5 to 9.0, or from 8.0 to 8.5. In this context, the isoelectric point of the CPP is the arithmetic mean of the isoelectric points of the amino acids forming the CPP.

In a specific embodiment of the present disclosure, the CPPs comprise between 2 to 19, between 3 to 16, between 4 to 14, and/or between 6 to 12 arginine moieties as well as one or more moieties selected from the group consisting of tyrosine, threonine, serine, lysine, aspartic acid, glutamic acid, glutamine, asparagine, and cysteine. For example, the CPP may comprise nine arginine moieties and one cysteine moiety in a ring system and are referred to as a cyclic cysteine R9 derivative. Another example is a cyclopeptide comprising nine arginine moieties and one lysine moiety in the ring system, which is referred to as cyclic R9K derivative.

The CPPs of this invention include dimerized CPPs, wherein homo- and heterodimers are within the scope of this disclosure. Dimerization of CPPs can be affected by any means known in the art. In a particular embodiment, CPPs are dimerized via the tripeptide KAK.

CPPs allow easy up-scaling, especially using solid phase synthesis techniques. CPPs are associated with membrane permeability and nucleolar accumulation.

In one embodiment the inhibitor may comprise nucleic acid covalently bound to a CPP and/or a carrier in a conjugate.

In one embodiment the inhibitor may comprise a miR-574-5p RNA antagomiR (SEQ ID No: 2 or 3) and/or miR-574-5p PNA antagomiR (SEQ ID No: 4 or 5) or even more truncated nucleic acid (SEQ ID No: 42) as outlined before. In one embodiment the inhibitor may comprise the nucleic acid covalently bound to CPP directly, in other embodiments by means of a PEG linker. The compounds can be bio-orthogonal covalently linked to CPP (i.e. "bio-orthogonal" being a chemical reaction that can occur inside of living systems without interfering with native biochemical processes). They allow for stronger hybridisation and increased half-life.

In one embodiment the disclosure pertains to an inhibitor which comprises PNA and is conjugated to the cell penetrating peptide (CPP) comprising SEQ IDs NO: 6-12, or 25-33, or 41, optionally, via a linker, for use in intra-articular treatment of a disease selected from the group consisting of osteoarthritis (OA), rheumatoid arthritis (RA), psoriatic arthritis, ankylosing spondylitis, gout, juvenile arthritis, systemic lupus erythematosus (SLE), Sjögren's syndrome and osteoporosis.

In one embodiment the linker may be a polyethylene-glycol (PEG)-linker. The PEG linker may have one or more ethylene glycol units. In one embodiment, the PEG linker contains one ethylene glycol unit, two or more ethylene glycol units or three or more ethylene glycol units. The solubility can be adjusted by suitable selection of the linker length.

In one embodiment the linker is a PEG-Linker, wherein the linker length may be chosen from 1 PEG-units (-CH₂-CH₂-O-) up to 175 PEG-units, i.e., with a molecular weight of between about 200 to about 35.000 g/mol. In one embodiment the PEG-Linker may have less than 10 units, less than 5 units, less than 3 units. In one embodiment the PEG-Linker may have from 1 to 20 units, from 2 to 15 units, or from 3 to 10 units.

In another embodiment the linker may be an AEEA-linker. AEEA (N-(2-aminoethyl)-ethane-1,2-diamine) is a bifunctional linker molecule containing amino groups at both ends. It is often used in bioconjugation applications due to its ability to form stable amide bonds with carboxyl groups.

Further, suitable linkers may be selected from the group consisting of PEG-linkers, AEEA-linker, glycine-serine linkers (linkers consisting of repeated glycine (Gly) and serine (Ser) residues), polyalanine linkers (polyalanine linkers consisting of repeated alanine (Ala) residues), coiled-coil linkers (linkers utilizing the natural coiled-coil motif found in proteins like leucine zippers to connect protein domains), helical linkers (linkers mimicking natural protein motifs such as α-helices), cysteine linkers (cysteine residues can be engineered into proteins for site-specific conjugation using thiol-reactive chemistries), thioether linkers (linkers utilising the thiol chemistry of cysteine residues to form stable linkages) and click chemistry linkers (linkers based on click chemistry reactions such as azide-alkyne cycloaddition).

The miR-574-5p inhibitor, in one embodiment the miR-574-5p antagonomiR, can be bound to lysine or a modified lysine. In one embodiment the C-terminal end of a PNA-antagomiR is bound to modified lysine. In one embodiment the modification of the lysine may consist in the presence of an amide group (-CONH₂) instead of the carboxyl group (-COOH). This may be advantageous for the synthesis of the PNA by solid phase synthesis. In addition, the solubility of the PNA may be increased.

In one embodiment the nucleic acid of the inhibitor is densely packed on the CPP and/or a carrier. This results in particularly good membrane permeability. In addition, the stability is increased. In one embodiment the surface density of the nucleic acid of a miR-574-5p inhibitor (such as comprising SEQ ID NO: 2, 3, 42, or, respectively, SEQ ID NO: 4 and 5) on the nanoparticles, may be at least 10¹⁰ molecules per cm², at least 10¹¹ molecules per cm², or at least 10¹² molecules per cm². In one embodiment, however, the surface density may be at most 10¹⁴ molecules per cm², at most 5*10¹³ molecules per cm², or at most 2*10¹³ molecules per cm². The surface density can of antagomiR oligonucleotides per 10 nm particle, may be in the range from 5 to 150 oligonucleotides, or in one embodiment from 10 to 70 oligonucleotides.

The present disclosure relates to an inhibitor of miR-574-5p (SEQ ID NO: 1) for use in intra-articular treatment of a disease selected from the group consisting of osteoarthritis (OA), rheumatoid arthritis (RA), psoriatic arthritis, ankylosing spondylitis, gout, juvenile arthritis, systemic lupus erythematosus (SLE), Sjögren's syndrome and osteoporosis.

In one embodiment the intra-articular treatment is selected from a direct injection into the joint or an arthroscopic lavage.

In one embodiment the inhibitor is additionally or independently conjugated to a binding protein which specifically binds to an osteoclast surface marker selected from the group consisting of tartrate-resistant acid phosphatase (TRAP), cathepsin K (cath K), calcitonin receptor (CTR), matrix metalloproteinase 9 (MMP9), and carbonic anhydrase II (CAII), as well as combinations thereof.

In one embodiment the inhibitor is additionally or independently embedded in and/or conjugated to the hull of an arthritogenic virus vector, optionally an alphavirus selected from the group consisting of chikungunya virus (CHIKV), o'nyong-nyong virus (ONNV), Ross River virus (RRV), Barmah Forest virus (BFV), Mayaro virus (MAYV), Sindbis virus (SINV) and Semliki Forest virus (SFV), as well as combinations thereof.

In one embodiment the inhibitor is additionally or independently embedded and/or conjugated to an intra-articular hyaluronic acid (IAHA).

In one embodiment the disclosure pertains to an antagomiR of any of the SEQ ID NO: 2, 3, 4, 5 or 42 for use in intra-articular treatment of a disease selected from the group consisting of osteoarthritis (OA), rheumatoid arthritis (RA), psoriatic arthritis, ankylosing spondylitis, gout, juvenile arthritis, systemic lupus erythematosus (SLE), Sjögren's syndrome and osteoporosis.

In yet a further embodiment the inhibitor of miR-574-5p may reduce the endogenous miR-574-5p expression levels to about 1%, to about 5%, to about 10%, to about 15%, to about 20%, or to about 25% of the normal endogenous miR-574-5p expression levels. In one embodiment the inhibitor of miR-574-5p may reduce the endogenous miR-574-5p expression levels from 1% to 25%, from 5% to 20%, or from 10% to 15% of the normal endogenous miR-574-5p expression levels. In one embodiment the inhibitor of miR-574-5p may reduce the endogenous miR-574-5p expression levels by at least 30%, at least 40%, at least 50%, at least 60%, at least 70%, at least 80%, or at least 90% as compared to the normal endogenous miR-574-5p expression levels.

In yet a further embodiment the inhibitor of miR-574-5p may relatively reduce the PGE2-level to about 0.0-fold, to about 0.1-fold, to about 0.2-fold, to about 0.3-fold, to about 0.4-fold, to about 0.5-fold, to about 0.6-fold, to about 0.7-fold, to about 0.8-fold, or to about 0.9-fold as compared to the normal PGE2-level.

In yet a further embodiment about 5 µM of inhibitor of miR-574-5p may relatively reduce the osteoclast number to about 0.1-fold, to about 0.2-fold, to about 0.3-fold, to about 0.4-fold, to about 0.5-fold, to about 0.6-fold, or to about 0.7-fold as compared to the normal osteoclast number.

In yet a further embodiment about 10 µM of inhibitor of miR-574-5p may relatively reduce the osteoclast number to about 0.05-fold, to about 0.06-fold, to about 0.07-fold, to about 0.08-fold, to about 0.09-fold, to about 0.1-fold, or to about 0.2-fold as compared to the normal osteoclast number.

In yet a further embodiment about 15 µM of inhibitor of miR-574-5p may relatively reduce the osteoclast number to about 0.0-fold, to about 0.005-fold, to about 0.01-fold, to about 0.02-fold, to about 0.03-fold, to about 0.04-fold, or to about 0.05-fold as compared to the normal osteoclast number.

In one embodiment the inhibitor of miR-574-5p may have an IC₅₀ of about 10 µM or less, IC₅₀ of about 7.5 µM or less, IC₅₀ of about 5 µM or less, IC₅₀ of about 3 µM or less, or IC₅₀ of about 2.5 µM or less.

Thus, in yet another embodiment the concentration of the inhibitor of miR-574-5p, for example in a pharmaceutical composition, may be at least 2.5 µM, at least 5 µM, at least 7.5 µM, at least 10 µM, at least 12.5 µM, at least 15 µM, at least 20 µM, at least 30 µM, at least 40 µM, or at least 50 µM.

In a further embodiment the inhibitor of miR-574-5p may reduce the pathology in a ACLT mouse model significantly. For example, the treatment with the inhibitor of miR-574-5p may result in an OARSI-score (overal joint) of 10 or less, of 9 or less, of 8 or less, of 7 or less, or of 6 or less. Further, additionally or independently, the treatment with the inhibitor of miR-574-5p may result in an OARSI-score (medial quadrants) of 5 or less, of 4 or less, of 3 or less, of 2 or less, or of 1 or less. Further, additionally or independently, the treatment with the inhibitor of miR-574-5p may result in an OARSI-score (lateral quadrants) of 5 or less, of 4 or less, of 3 or less, of 2 or less, or of 1 or less. Further, additionally or independently, the treatment with the inhibitor of miR-574-5p may result in an OARSI-score (tibial quadrants) of 4 or less, of 3 or less, of 2 or less, or of 1 or less. Further, additionally or independently, the treatment with the inhibitor of miR-574-5p may result in an OARSI-score (femoral quadrants) of 7 or less, of 6 or less, of 5 or less, of 4 or less, or of 3 or less.

In a further embodiment the treatment with the inhibitor of miR-574-5p may result in an osteophyte score of 2 or less, or of 1 or less.
- Wherein the osteophyte score is graded as follows:
   ∘ Grade 0: Absent osteophytes:
      ▪ No evidence of bony outgrowths at the joint margins.
      ▪ Joint space appears smooth and uniform on imaging.
   ∘ Grade 1: Mild osteophytes:
      ▪ Small, isolated osteophytes are present.
      ▪ Minimal or no joint space narrowing.
      ▪ Osteophytes may be barely perceptible or visible only on certain imaging views.
   ∘ Grade 2: Moderate osteophytes:
      ▪ Multiple or larger osteophytes are present.
      ▪ Moderate joint space narrowing, indicating mild to moderate cartilage loss.
      ▪ Osteophytes may be more prominent and clearly visible on imaging.
   ∘ Grade 3: Severe osteophytes:
      ▪ Large, prominent osteophytes are present, often extending into the joint space.
      ▪ Marked joint space narrowing, indicating significant cartilage loss and joint degeneration.
      ▪ Osteophytes may contribute to joint deformity, instability, or functional impairment.

In a further embodiment the treatment with the inhibitor of miR-574-5p may result in a meniscal score of 2 or less, or of 1 or less.
- Wherein the "meniscal score" is graded according to the International Society of Arthroscopy, Knee surgery and Orthopaedic Sports medicine (ISAKOS) classification:
   ∘ Grade I: Normal meniscus.
   ∘ Grade II: Meniscal tear with stable margins.
   ∘ Grade III: Meniscal tear with unstable margins.
   ∘ Grade IV: Complete meniscal extrusion.

In a further embodiment the treatment with the inhibitor of miR-574-5p may result in a synovitis score of 2 or less, or of 1 or less.
- Wherein the synovitis score is graded as follows:
   ∘ Grade 0: Absent or minimal synovitis:
      ▪ No detectable inflammation of the synovial membrane.
      ▪ No joint effusion (fluid accumulation).
   ∘ Grade 1: Mild synovitis:
      ▪ Minimal thickening of the synovial membrane.
      ▪ Mild joint effusion, if present.
      ▪ Mild tenderness or discomfort upon palpation of the joint.
   ∘ Grade 2: Moderate synovitis:
      ▪ Moderate thickening of the synovial membrane.
      ▪ Moderate joint effusion.
      ▪ Increased warmth and tenderness of the joint.
      ▪ Mild limitation of joint motion.
   ∘ Grade 3: Severe synovitis:
      ▪ Marked thickening of the synovial membrane.
      ▪ Large joint effusion, potentially causing joint swelling and deformity.
      ▪ Severe tenderness, warmth, and pain.
      ▪ Significant limitation of joint motion, potentially leading to joint stiffness and functional impairment.

In a further embodiment the treatment with the inhibitor of miR-574-5p may result in a ligament score of grade 2 or less, or of grade 1 or less.
- Wherein ligament score "Grade I" is defined as mild ligament sprain, involving minimal stretching or microscopic tearing of the ligament fibres. There may be mild pain and swelling, but joint stability is generally preserved.
- Wherein ligament score "Grade II" is defined as moderate ligament sprain, involving partial tearing of the ligament fibres. There is typically more pain, swelling, and bruising, and joint instability may be present.
- Wherein ligament score "Grade III" is defined as severe ligament sprain or complete ligament tear. This is the most serious type of ligament injury, involving complete disruption of the ligament fibres. There is often significant pain, swelling, bruising, and joint instability, and the joint may be unable to bear weight or move normally.

In a further embodiment the treatment with the inhibitor of miR-574-5p may result in a CTX-II-level (in pg/ml) of less than 6, less than 5, less than 4, or less than 3. In a further embodiment the treatment with the inhibitor of miR-574-5p may result in a CTX-II-level (in pg/ml) from 1.0 to 6.0, from 1.5 to 5.0, or from 2.0 to 4.0.

Further, the treatment of monocytes with the inhibitor of miR-574-5p inhibits osteoclast maturation. Further, the treatment of macrophages with the inhibitor of miR-574-5p inhibits osteoclast maturation.

In a further embodiment the above-mentioned inhibitor may comprise a sequence selected from SEQ ID NO: 2, 3, 4, 5 or 42.

According to the invention, the extent of osteoclast differentiation may be used as a measure of the extent of bone resorption. The effects of a substance on osteoclast differentiation are well known to those skilled in the art. Particularly suitable methods are also described in detail in the present example section. In summary, it is advantageous if osteoclast differentiation is induced in CD14* monocytes or M2-like macrophages with the aid of miR-574-5p in the presence or absence, respectively, of the respective potentially inhibitory substance to be tested, whereby the desired inhibitory effect can be determined if a significantly lower osteoclast differentiation is observed in the presence of the substance to be tested compared to the absence of the substance. Other test methods are also possible. For example, the extent of osteoclast differentiation and/or the extent of bone resorption can alternatively or additionally be investigated in an animal model, in particular an arthritis mouse model such as CIA (collagen-induced arthritis).

The pharmaceutical composition contains carriers selected from the group consisting of intra-articular hyaluronic acid (IAHA), liposomes, nanocarriers (NCs) and/or cholesterol. In one embodiment the carriers may be NCs.

In one embodiment the NC are not nanoparticles (NPs), especially iron oxide nanoparticles, gold nanoparticles and/or silver nanoparticles are excluded from this disclosure due to unwanted side-effects.

The present invention also relates to a process for preparing a pharmaceutical composition of the invention comprising the following steps:
a) Providing a miR-574-5p inhibitor according to the invention (SEQ ID NOs: 2-5),
b) Coupling of the miR-574-5p inhibitor to cell-penetrating peptides (CPPs) (SEQ ID NOs: 6-12, or 25 - 33, or 41) and/or intra-articular hyaluronic acid (IAHA), liposomes, nanocarriers (NCs) and/or cholesterol.

In one embodiment the coupling according to step b) is performed via click chemistry, for example as described in Cutler J. I., Zheng D., Xu X., Giljohann D. A., Mirkin C. A. (2010), "Polyvalent oligonucleotide mono oxide nanoparticle "click" conjugates." Nano Letters 10: 1477-1480. In one embodiment CPPs and/or carriers may be present in aminated form are reacted with azido butyrate or azido acetate to yield a free azide group. For example, the azide group may bond with modified nucleotides, in one embodiment with a 1:1 stoichiometry. As described above, the miR- 574-5p inhibitors according to the invention may be or comprise RNA or RNA-analoga, so that these molecules can be coupled particularly well via correspondingly modified nucleotides, in particular at the 5' end or at the 3' end (in one embodiment at the 5' end), to correspondingly CPPs or other molecules. One nucleotide modification in this context may be a terminal alkyne group, especially at the 5' end of the nucleotide.

The coupling according to step b) to CPPs may be carried out by means of a PEG linker. It is possible in the context of the present invention to synthesize CPP and miR-574-5p inhibiting RNA-or RNA-analogue (such as an antagomiR) separately and then to couple them together. In one embodiment the entire construct may be built up monomer by monomer in a continuous manner. In some embodiments the whole CPP-RNA and/or CPP-PNA construct is called an "inhibitor" and may be built starting with the C-terminal PNA monomer (or the even more C-terminal modified lysine) and ending with the N-terminal amino acid of the CPP part.

The present disclosure also relates to the use of a miR-574-5p inhibitor as an intra-articular medicament, and/or for the intra-articular treatment of chronic joint diseases, in particular osteoarthritis and other arthritic diseases. In one embodiment the present disclosure relates to the use for the treatment of reducing bone resorption in chronic joint diseases selected from the group consisting of rheumatoid arthritis (RA), psoriatic arthritis, ankylosing spondylitis, gout, juvenile arthritis, systemic lupus erythematosus (SLE), Sjögren's syndrome and osteoporosis.

### SHORT DESCRIPTION OF THE FIGURES

Figure 1: Design of a miR-574-5p inhibitor according to the present disclosure (SEQ ID NO: 5 and SEQ ID NO: 6) as well as the CPP-control (SEQ ID NO: 6) and the CPP-nucleic-acid control (comprising SEQ ID NO:6 and SEQ ID NO: 13).

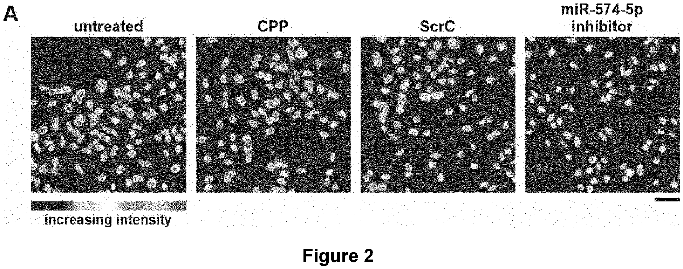

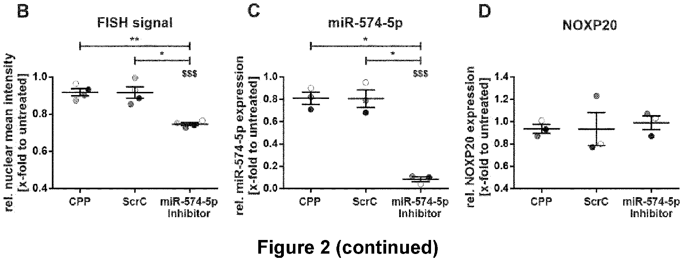

Figure 2: MiR-574-5p inhibitor reduces endogenous miR-574-5p levels as visualized by reduced fluorescence (A) & (B), reduced MiR-574-5p expression (C) and unaltered NOXP20 expression (D).

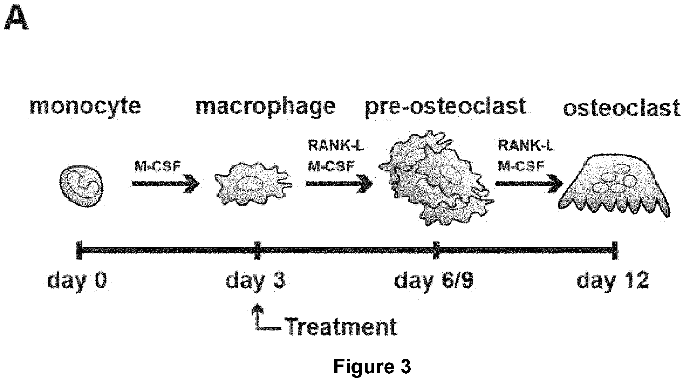

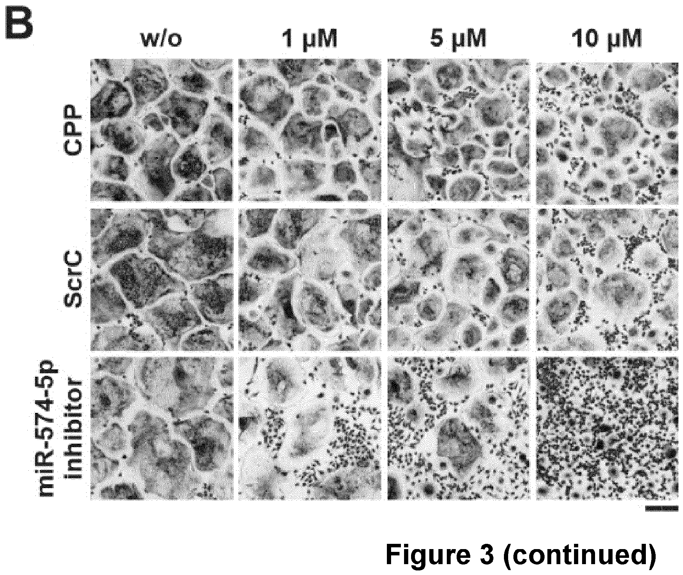

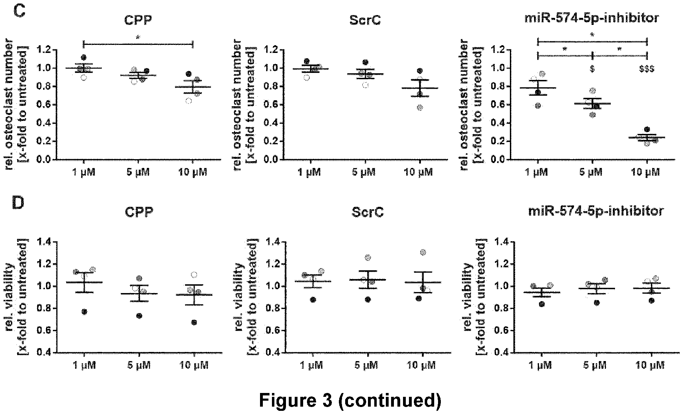

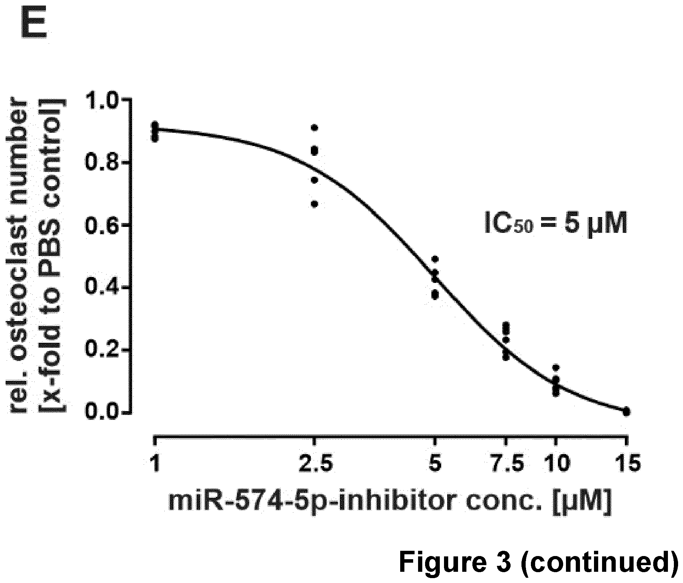

Figure 3: MiR-574-5p inhibitor inhibits osteoclast maturation. (A) shows a schematic of normal osteoclast differentiation (treatment with inhibitor on day 3, i.e. macrophage state); (B) shows the inhibition by light microscope; (C) the relative osteoclast number vs. increasing concentrations of controls and inhibitor, showing that only the inhibitor shows a clear concentration-dependent effect; (D) the relative viability of the cells, showing that the inhibitor is non-toxic to the cells; (E) depicts the IC50 of inhibitor concentration vs. osteoclast inhibition (at 5 µM).

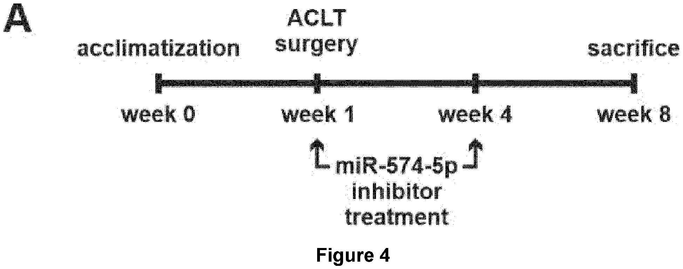

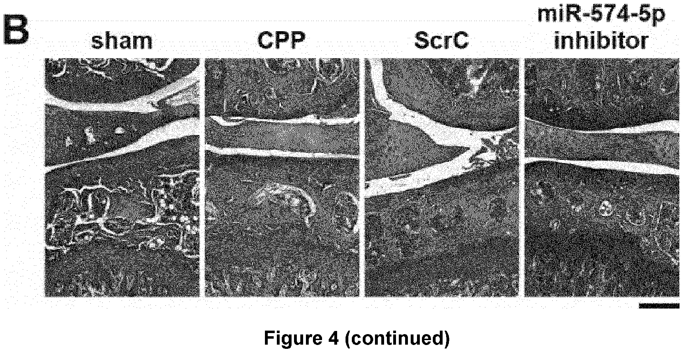

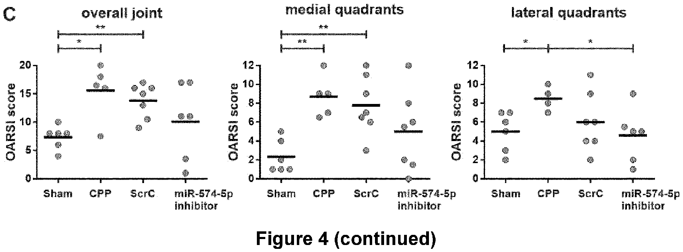

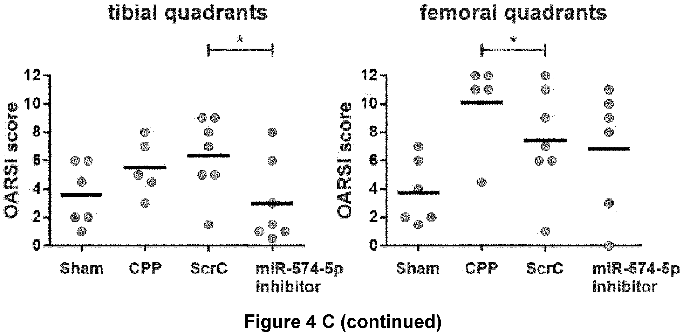

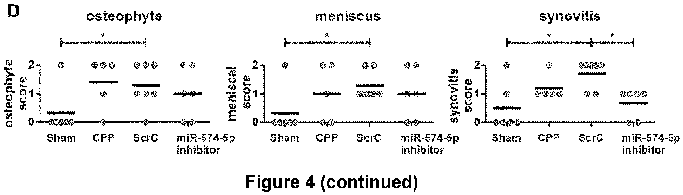

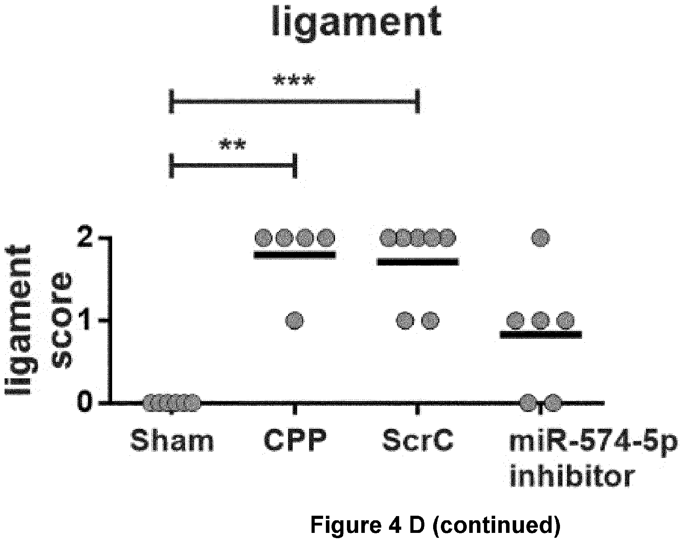

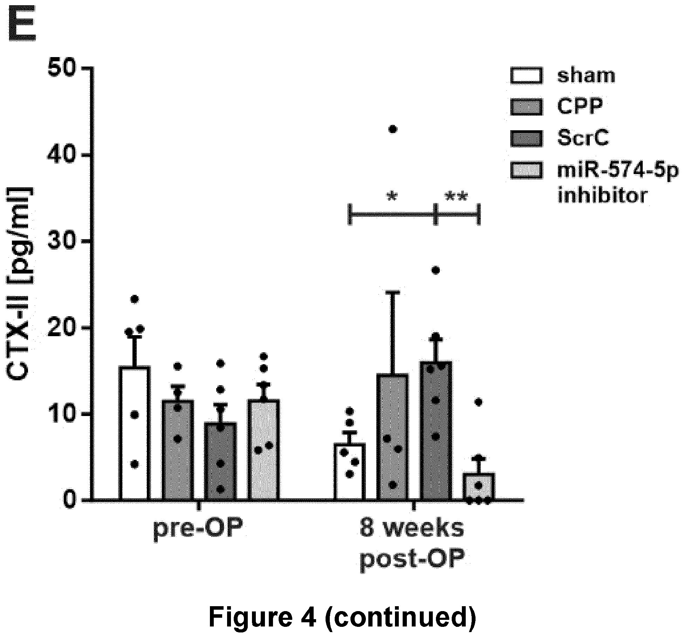

Figure 4: MiR-574-5p inhibitor reduces pathology in ACLT mouse model. (A) shows the schematics of the animal experiment; (B) depicts safranin O stained tissue sections; (C) shows OARSI-scores of inhibitor vs. control treated mice; (D) shows osteophyte, meniscus, synovits and ligament scores of inhibitor vs. controls; (E) shows CTX-II -values pre and post OP of inhibitor vs. controls.

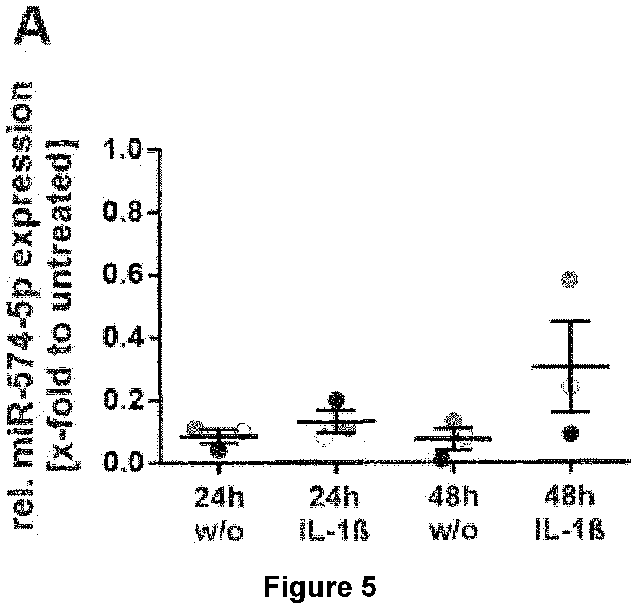

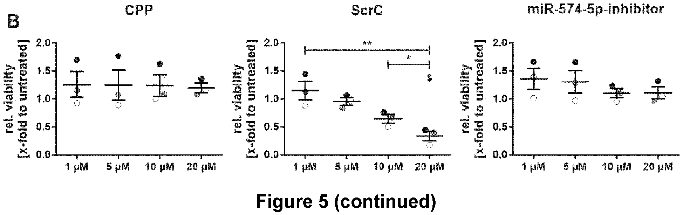

Figure 5: Long lasting miRNA inhibition and additional HepG2 cytotoxicity. (A) shows even 48h after treatment a miR-574-5p inhibition; whereas in (B) the long-term relative viability is not impaired by the inhibitor (but by the SrcC-control).

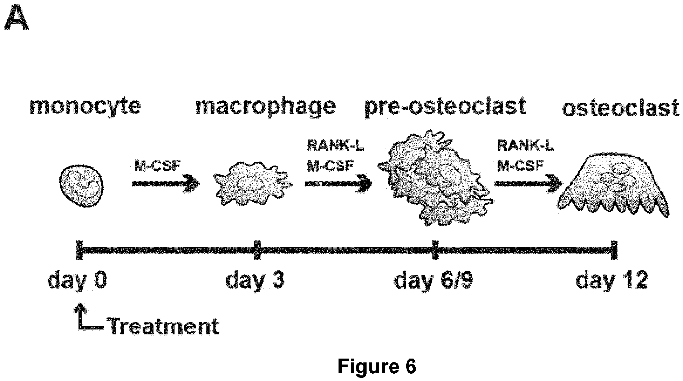

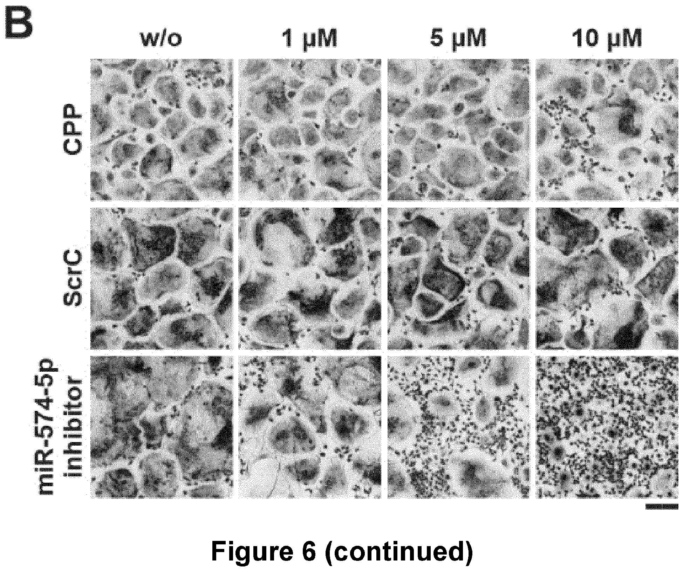

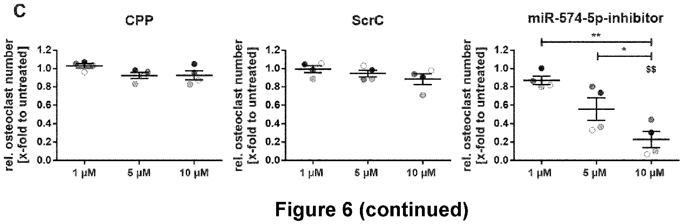

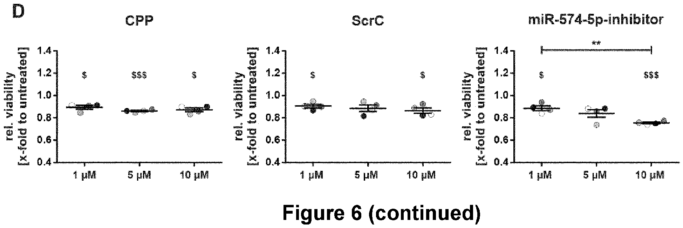

Figure 6: MiR-574-5p inhibitor treatment of monocytes (treatment on day 0) inhibits osteoclast maturation. (A) shows a schematic of normal osteoclast differentiation (treatment with inhibitor on day 0, i.e. monocyte state); (B) shows the inhibition by light microscope; (C) the relative osteoclast number vs. increasing concentrations of controls and inhibitor, showing that only the inhibitor shows a clear concentration-dependent effect; (D) the relative viability of the cells, showing that the inhibitor is non-toxic to the cells.

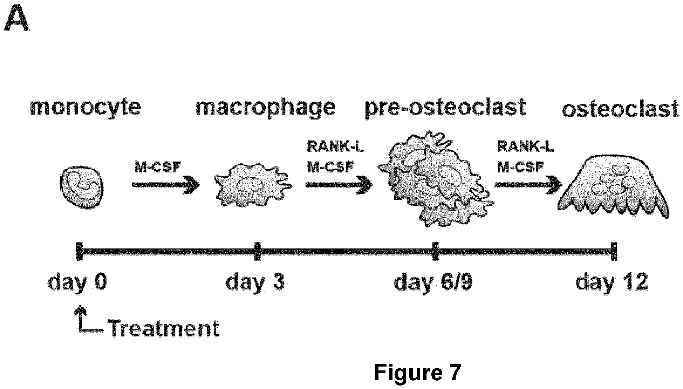

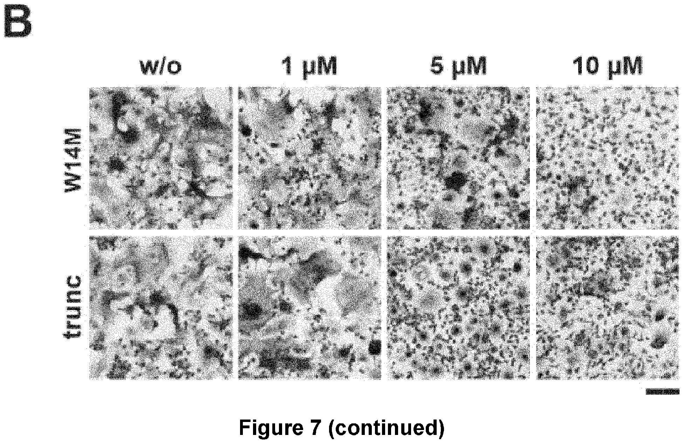

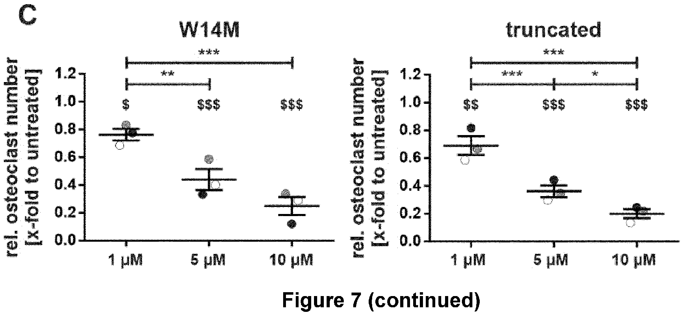

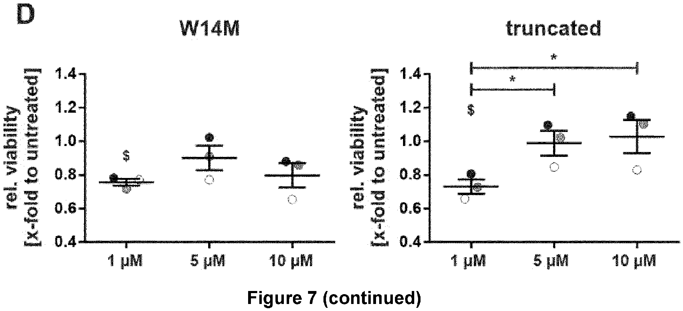

Figure 7: Modified miR-574-5p inhibitor treatment of monocytes (treatment on day 0) inhibits osteoclast maturation. (A) shows a schematic of normal osteoclast differentiation (treatment with inhibitors W14M (SEQ ID No: 41) and truncated (SEQ ID No: 42) on day 0; (B) shows the inhibition by confocal microscope; (C) the relative osteoclast number vs. increasing concentrations of controls and inhibitor, showing that only the inhibitor shows a clear concentration-dependent effect; (D) the relative viability of the cells, showing that the inhibitor is non-toxic to the cells.

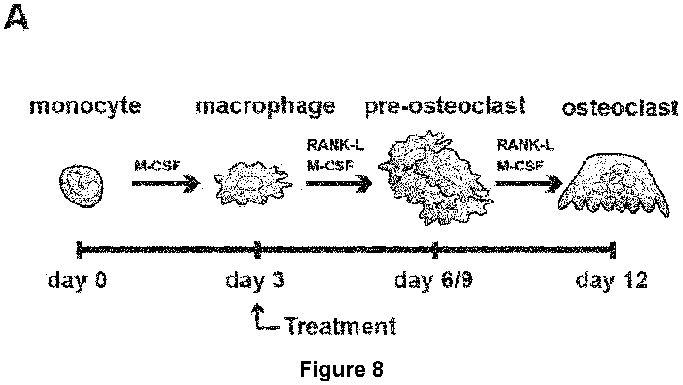

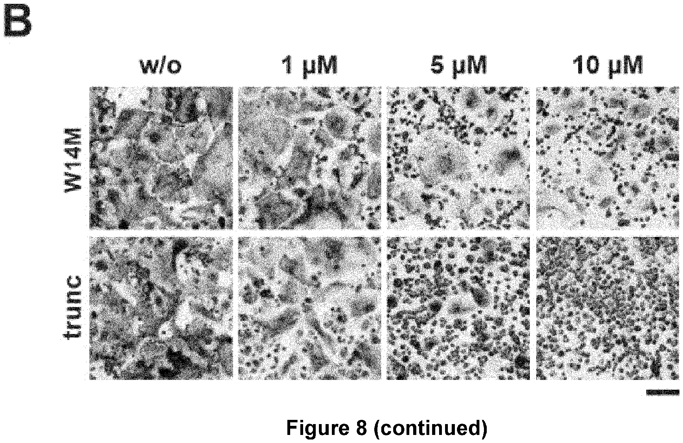

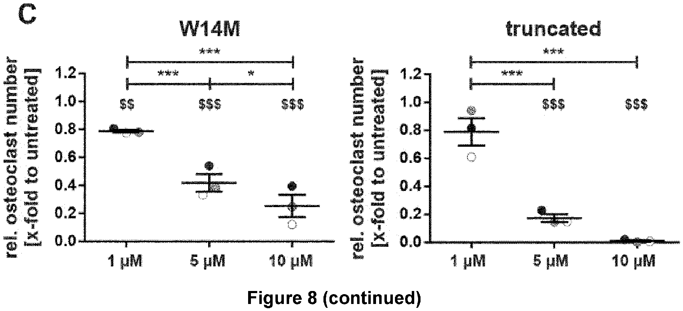

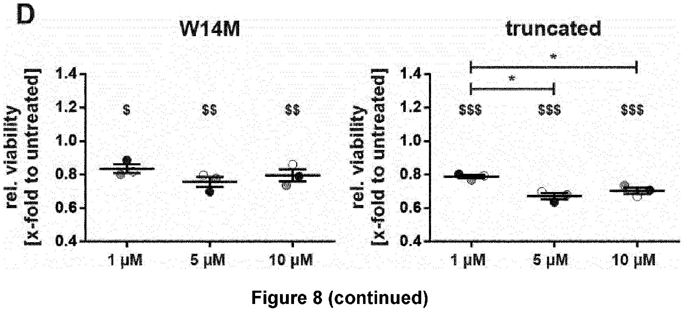

Figure 8: Modified miR-574-5p inhibitor treatment of macrophages (treatment on day 3) inhibits osteoclast maturation. (A) shows a schematic of normal osteoclast differentiation (treatment with inhibitors W14M (SEQ ID No: 41) and truncated (SEQ ID No: 42) on day 3; (B) shows the inhibition by light microscope; (C) the relative osteoclast number vs. increasing concentrations of controls and inhibitor, showing that only the inhibitor shows a clear concentration-dependent effect; (D) the relative viability of the cells, showing that the modified inhibitors express higher cytotoxicity compared to the unmodified inhibitor.

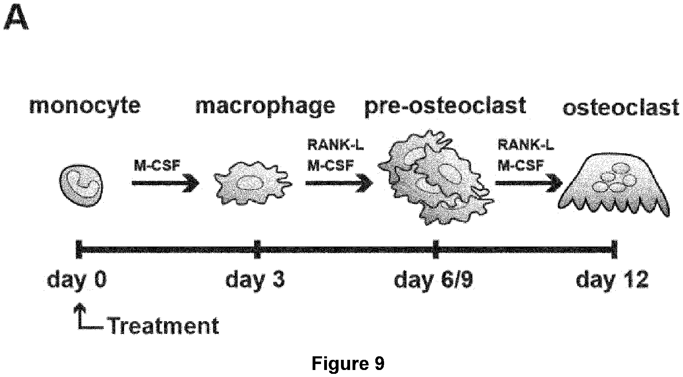

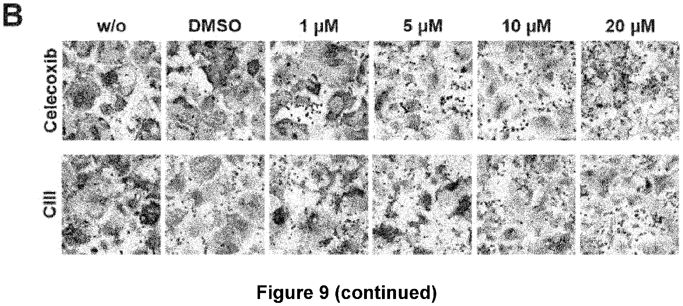

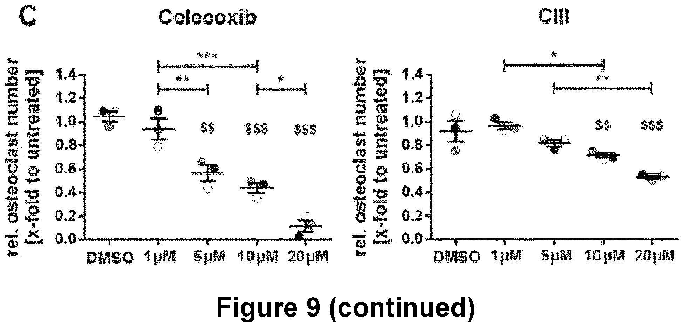

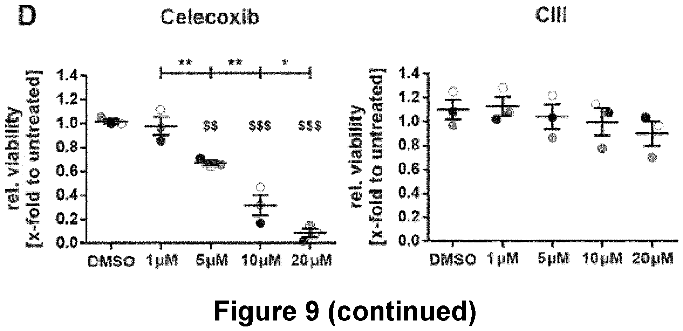

Figure 9: Prostanoid pathway inhibitor treatment of monocytes (treatment on day 0) influences osteoclast maturation. (A) shows a schematic of normal osteoclast differentiation (treatment on day 0); (B) shows the inhibition by light microscope; (C) the relative osteoclast number vs. increasing concentrations of prostanoid pathway inhibitors; (D) the relative viability of the cells, showing that the prostanoid pathway inhibitor celecoxib is toxic to the cells.

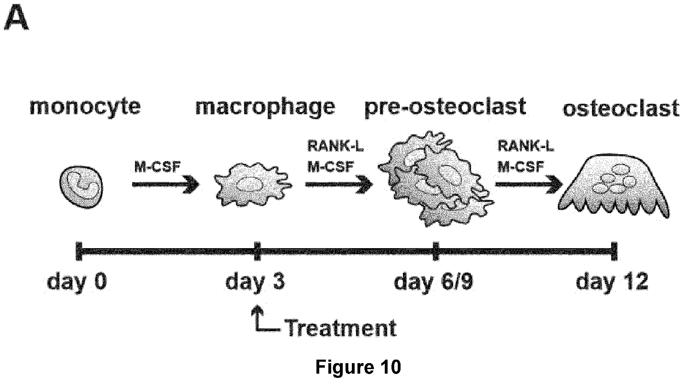

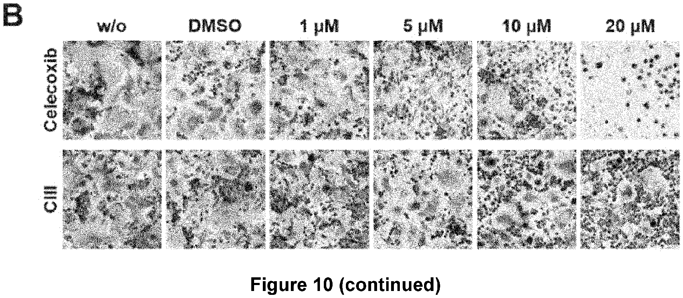

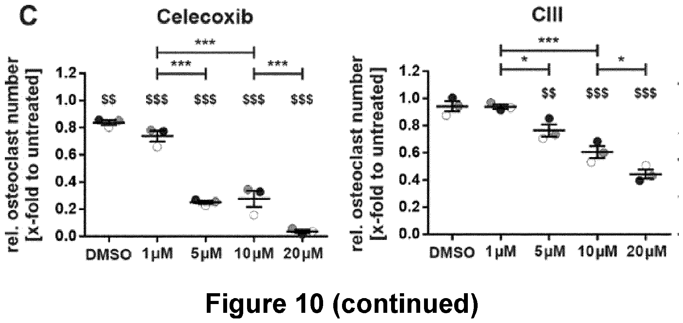

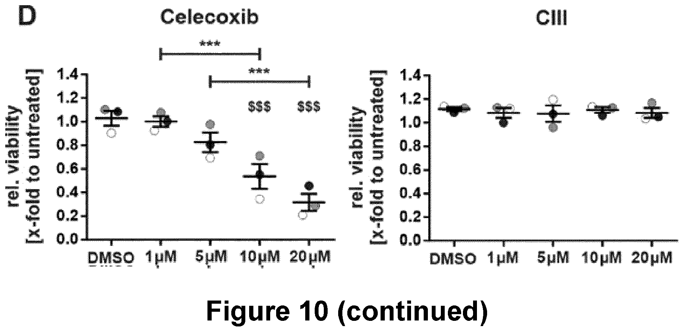

Figure 10: Prostanoid pathway inhibitor treatment of macrophages (treatment on day 3) influences osteoclast maturation. A) shows a schematic of normal osteoclast differentiation (treatment on day 3); (B) shows the inhibition by confocal microscope; (C) the relative osteoclast number vs. increasing concentrations of prostanoid pathway inhibitors; (D) the relative viability of the cells, showing that the prostanoid pathway inhibitor celecoxib is toxic to the cells.

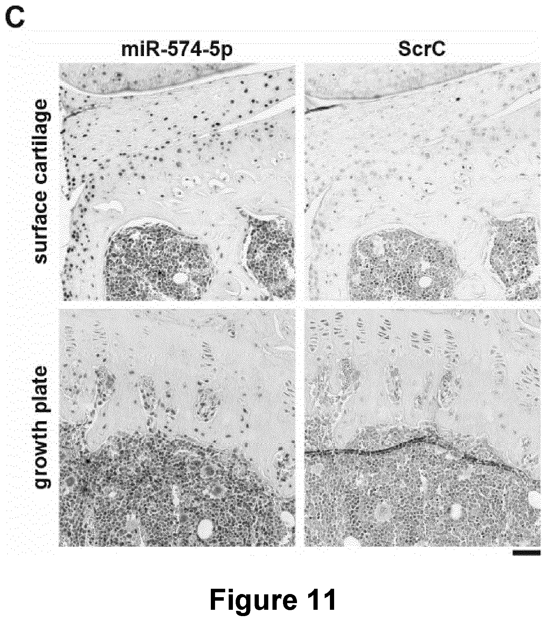

Figure 11: MiR-574-5p ISH of frontal knee sections, showing the expression of miR-574-5p within the joint.

### DEFINITIONS and SEQUENCES

### Definitions

The term "inhibitor" or "inhibitor of miR-574-5p (SEQ ID NO: 1)" defines any molecule able to inhibit the biologically function of miR-574-5p of SEQ ID NO: 1. An inhibitor of miR-574-5p targets both its intracellular function as a regulator of prostaglandin synthesis and its extracellular function as a facilitator of osteoclast genesis.

In one embodiment this may be a nucleic acid molecule at least partially complementary to miR-574-5p of SEQ ID NO: 1 as further explained hereinunder. In further embodiments the inhibitor is based on a peptide nucleic acid (PNA) sequence conjugated to a cell-penetrating peptide (CPP). In one embodiment a miR-574-5p inhibitor comprising or consisting of peptidic nucleic acid (PNA). In one embodiment it may be a PNA-based antagomiR against miR-574-5p.

"Intra-articular treatment" refers to a medical procedure in which medication or therapeutic agents are brought into a joint by direct injection or arthroscopic lavage. This approach is commonly used to treat various joint-related conditions, particularly those associated with inflammation, pain, and reduced joint function. Intra-articular treatments can be used for both diagnostic and therapeutic purposes, and they are often performed by healthcare professionals, such as rheumatologists, orthopedic surgeons, or pain management specialists. Key aspects of intra-articular treatment comprise:
In case of a "direct injection into the joint", the healthcare provider uses a sterile needle and syringe to inject the medication, for example a pharmaceutical composition comprising the inhibitor of miR-574-5p (SEQ ID NO: 1), directly into the affected joint.

In some cases, intra-articular injections are used for diagnostic purposes. By injecting a local anesthetic into the joint, the healthcare provider can determine if the joint is the source of a patient's pain. If the pain is significantly reduced or eliminated after the injection, it suggests that the joint is indeed the cause of the discomfort.

Intra-articular injections are frequently administered to provide therapeutic relief for joint-related conditions. Depending on the specific treatment and the patient's response, intra-articular injections may be administered as a one-time treatment or as a series of injections over time. The frequency and number of injections can vary.

Intra-articular treatment offers several advantages. It delivers medication directly to the affected area, which can result in faster and more targeted relief. It also minimizes systemic side effects compared to oral medications, as the medication is concentrated at the site of the problem. Systemic effects can be minimized.

Intra-articular treatment is often an integral part of the management of joint-related conditions, and its effectiveness can vary depending on the specific condition, the choice of medication, and the patient's individual response. In one embodiment "intra-articular treatment" refers to an intra-articular injection. Intra-articular injections, which involve delivering medication directly into a joint space, offer several advantages compared to systemic (oral or intravenous) treatments, particularly in the context of managing joint-related conditions such as osteoarthritis or inflammatory arthritis. Some advantages include:
- Localized effect: Intra-articular injections deliver medication directly to the affected joint, allowing for a more targeted and concentrated therapeutic effect. This localized delivery reduces the risk of systemic side effects associated with oral or intravenous medications.
- Enhanced efficacy: By delivering medication directly to the site of pathology, intra-articular injections can achieve higher concentrations of the drug within the joint, potentially leading to improved efficacy compared to systemic treatments.
- Reduced systemic side effects: Because intra-articular injections minimize systemic exposure to the medication, they are associated with fewer systemic side effects such as gastrointestinal upset, cardiovascular effects, or effects on other organs that can occur with oral or intravenous medications.
- Rapid onset of action: Intra-articular injections can provide more rapid relief of symptoms compared to systemic treatments, as the medication acts directly at the site of inflammation or pain.
- Decreased need for high doses: Since the medication is delivered directly to the affected joint, lower doses may be needed compared to systemic treatments, reducing the risk of adverse effects associated with higher doses.
- Avoidance of first-pass metabolism: With systemic treatments, medications must pass through the gastrointestinal tract or liver before reaching systemic circulation, which can result in degradation or alteration of the drug's efficacy. Intra-articular injections bypass this first-pass metabolism, ensuring more reliable delivery of the medication to the joint.
- Alternative for patients with contraindications to systemic treatments: Some patients may have contraindications to systemic medications due to comorbidities or medication interactions. In such cases, intra-articular injections may provide a viable alternative for managing joint-related symptoms.

"Arthroscopic lavage" is a surgical procedure used to clean and irrigate the interior of a joint, typically the knee, shoulder, or hip, using minimally invasive arthroscopic techniques. This procedure is often performed by orthopedic surgeons and is used to diagnose and treat certain joint conditions. Key aspects of arthroscopic lavage comprise:

### Procedure:

- Preparation: The patient is typically placed under anesthesia, either general or regional anesthesia, to ensure they are pain-free and relaxed during the procedure.
- Incisions: Small incisions, typically about the size of a buttonhole, are made near the joint that needs to be examined and treated. These incisions are used for the insertion of an arthroscope (a thin, flexible tube with a camera at the end) and surgical instruments.
- Visualization: The arthroscope is inserted into one of the incisions, providing the surgeon with a clear view of the joint's interior on a monitor. This real-time visual feedback allows the surgeon to assess the joint's condition.
- Irrigation and Lavage: Once the joint is visualized, the surgeon uses another small incision to introduce sterile saline solution or a similar fluid into the joint space. The fluid may comprise a pharmaceutical composition comprising the inhibitor of miR-574-5p. This fluid helps to wash out debris, inflammatory particles, and damaged tissue, essentially "laundering" the joint. The process of flushing out the joint with this fluid is known as lavage.
- Evaluation: While irrigating and lavaging the joint, the surgeon may also use surgical instruments to gently remove loose tissue, cartilage, or other debris. This helps improve the overall condition of the joint and may provide therapeutic benefits.
- Closure: After the procedure is complete, the incisions are closed with stitches or adhesive strips, and a sterile dressing is applied.

### Purposes:

Arthroscopic lavage serves several purposes:
- Diagnostic: It can be used to assess the condition of a joint, identify the cause of pain or dysfunction, and make an accurate diagnosis. The arthroscope allows the surgeon to directly visualize the joint's interior and identify any abnormalities, such as torn ligaments, cartilage damage, or inflammation.
- Therapeutic: In some cases, arthroscopic lavage can provide therapeutic benefits by removing inflammatory substances and debris from the joint, potentially reducing pain and improving joint function. This is especially common in cases of osteoarthritis.
- Preoperative Planning: Arthroscopic lavage can be used as a part of preoperative planning for more extensive joint surgeries. It helps the surgeon understand the joint's condition and plan the appropriate treatment.

Arthroscopic lavage is a minimally invasive procedure that typically results in less postoperative pain and a quicker recovery compared to open surgical techniques. However, its effectiveness can vary depending on the underlying joint condition.

A "nucleic acid-based inhibitor" is a type of therapeutic agent designed to target and interfere with the function of specific genes or gene products (such as RNA or proteins) within a cell. These inhibitors are typically made from nucleic acids, which are the building blocks of genetic information in living organisms. There are two main types of nucleic acid-based inhibitors: antisense oligonucleotides and small interfering RNAs (siRNAs).

In the context of nucleic acids, "complementary" refers to the specific base-pairing interactions between the nitrogenous bases of two nucleic acid strands. Nucleic acids are composed of long chains of repeating units called nucleotides, and there are four different nitrogenous bases in nucleic acids:
- Adenine (A)
- Thymine (T) - In DNA only
- Cytosine (C)
- Guanine (G)

In DNA, adenine (A) always pairs with thymine (T), and cytosine (C) always pairs with guanine (G). This pairing is based on hydrogen bonding interactions between the bases:
- Adenine (A) forms two hydrogen bonds with thymine (T).
- Cytosine (C) forms three hydrogen bonds with guanine (G).

This complementary base-pairing is essential for the structure and function of DNA, where two complementary DNA strands come together to form the famous double helix.

In RNA, which is closely related to DNA but has uracil (U) instead of thymine (T), the base-pairing rules are as follows:
- Adenine (A) pairs with uracil (U) in RNA, similar to how it pairs with thymine (T) in DNA.
- Cytosine (C) pairs with guanine (G) in RNA, just as it does in DNA.

The skilled person will know that the disclosed nucleic acid sequences with thymine may comprise partially and/or fully uracil instead, and vise-versa.

The complementary base-pairing is a fundamental principle in molecular biology and genetics. It allows for the replication of DNA, transcription of DNA into RNA, and translation of RNA into proteins, all of which are essential processes in the central dogma of molecular biology.

When one nucleic acid sequence is disclosed as being complementary to another, it means that the sequence of bases in one strand is precisely matched to the sequence of bases in the other strand according to the base-pairing rules. This complementarity is crucial for the stability and specificity of nucleic acid interactions and plays a fundamental role in various biological processes, such as DNA replication, RNA synthesis, and gene expression.

According to the present disclosure, the nucleic acid comprised in the inhibitor may be at least 90%, at least 91%, at least 92%, at least 93%, at least 94%, at least 95%, at least 96%, at least 97%, at least 98%, at least 99%, at least 99,5%, at least 99,8%, or at least 99,9% complementary to miR-574-5p (SEQ ID NO: 1). In one embodiment the nucleic acid-based inhibitor may be up to 100%, up to 99,5%, up to 99%, up to 98%, or up to 95% complementary to miR-574-5p (SEQ ID NO: 1). In one embodiment the nucleic acid-based inhibitor may be from 90% to 99%, from 92% to 98%, or from 95 to 97% complementary to miR-574-5p (SEQ ID NO: 1).

"Deoxyribonucleic acid", commonly known as DNA, is a molecule found in the cells of living organisms. It serves as the genetic blueprint or genetic code for the development, functioning, growth, and reproduction of all known living organisms, including animals, plants, bacteria, and viruses. DNA carries the hereditary information that is passed from one generation to the next.

"Ribonucleic acid", commonly known as RNA, is a molecule found in cells that plays several important roles in the transmission of genetic information and the synthesis of proteins. RNA is closely related to DNA (deoxyribonucleic acid), which carries the genetic code in most organisms.

"Peptide nucleic acid (PNA)" is a synthetic molecule that combines features of both nucleic acids (DNA and RNA) and peptides (amino acids). PNA was first developed in the 1990s as a novel type of nucleic acid analog with unique properties. Unlike DNA and RNA, PNAs have a neutral backbone made of repeating N-(2-aminoethyl)-glycine units, which makes them resistant to enzymatic degradation and allows for stable hybridization with complementary DNA and RNA sequences.

"Locked nucleic acid (LNA)" is a modified nucleic acid analog that is used in molecular biology and genetics research. LNAs are synthetic RNA or DNA molecules with a modified ribose sugar ring that is "locked" into a rigid, conformationally constrained structure, enhancing their stability and specificity when binding to complementary RNA or DNA sequences.

"Morpholino oligonucleotides", often referred to as simply "morpholinos," are synthetic molecules used in molecular biology and genetics research to modify gene expression and function. Morpholinos are designed to specifically bind to and block the activity of RNA molecules, such as messenger RNA (mRNA), in a highly targeted manner. They are named after their characteristic morpholino rings, which replace the sugar-phosphate backbone found in natural nucleic acids like DNA and RNA.

"Peptide-oligonucleotide hybrids (PONHs)" are synthetic molecules that combine two distinct components: peptides and oligonucleotides. These hybrids are designed for various applications in molecular biology, biotechnology, and therapeutics. They merge the specificity of peptides (amino acid sequences) with the information-carrying capabilities of oligonucleotides (DNA or RNA sequences).

"Xeno-nucleic acid (XNA)" is a synthetic form of nucleic acid that differs from natural DNA and RNA in its chemical structure. XNAs are designed by chemists and researchers to have novel backbone structures while still retaining the ability to store and transmit genetic information. They are part of the broader field of synthetic biology and have potential applications in genetic research, biotechnology, and medicine.

"Aptamers" are short, single-stranded DNA or RNA molecules, or sometimes synthetic nucleic acid analogs, that can bind to specific target molecules with high affinity and specificity. They are often referred to as "chemical antibodies" because they can recognize and bind to a wide range of target molecules, including proteins, small molecules, and even whole cells.

"AntagomiRs" are synthetic molecules designed to inhibit the activity of microRNAs (miRNAs) in cells. MicroRNAs are small RNA molecules that play a crucial role in regulating gene expression by binding to messenger RNAs (mRNAs) and preventing their translation into proteins or facilitating their degradation. Antagomirs are used in molecular biology and biomedical research to study the functions of specific miRNAs and have potential therapeutic applications in diseases where miRNA dysregulation is implicated.

A "cell-penetrating peptide (CPP)", also known as a protein transduction domain (PTD) or a Trojan peptide, is a short peptide sequence composed of amino acids that has the unique ability to penetrate cell membranes and facilitate the delivery of various cargoes, including proteins, nucleic acids (such as DNA or RNA), drugs, and nanoparticles, into the interior of cells. This property makes CPPs valuable tools in biomedical research and drug development for targeted intracellular delivery.

Key characteristics of cell-penetrating peptides include:
- Membrane Penetration: CPPs have the ability to traverse cellular membranes, including the lipid bilayer of the cell membrane, which is typically impermeable to large molecules and charged molecules. This enables them to deliver cargo into the cytoplasm or other cellular compartments.
- Diverse Cargo Compatibility: CPPs can transport a wide range of cargo types, including therapeutic molecules (e.g., drugs, siRNA, peptides), imaging agents, and biomolecules (e.g., proteins, peptides, antibodies).
- Non-Toxicity: Many CPPs are relatively non-toxic to cells, making them suitable for use in various biological and medical applications.
- Versatility: CPPs can be chemically modified and engineered to improve their stability, specificity, and cargo delivery efficiency.
- Cellular Uptake Mechanisms: The exact mechanisms of CPP-mediated cellular uptake are still an active area of research and may vary among different CPPs. Some proposed mechanisms include endocytosis, direct translocation through the lipid bilayer, or a combination of mechanisms.

Examples of CPPs include:
- Tat peptide: Derived from the HIV-1 Tat protein, this CPP has been widely used for intracellular delivery of various cargoes.
- Penetratin: A synthetic CPP derived from the homeodomain of the Antennapedia transcription factor in Drosophila.
- Arginine-rich peptides: Peptides rich in arginine residues, such as poly-arginine peptides, have CPP properties.
- Transportan: A chimeric peptide derived from the neuropeptide galanin and the antimicrobial peptide mastoparan.

Applications of CPPs include drug delivery, gene therapy, protein therapy, and intracellular imaging. They have the potential to improve the effectiveness of therapeutic interventions by ensuring that drugs or genetic material reach their intracellular targets.

"Tartrate-resistant acid phosphatase (TRAP)", also known as acid phosphatase 5 (ACP5), is an enzyme found in various tissues and cells in the human body. TRAP is a metalloenzyme, which means it requires metal ions, such as zinc or iron, for its catalytic activity. It is primarily known for its role in bone metabolism and as a marker for certain cell types.

"Cathepsin K", often abbreviated as Cath K, is a lysosomal cysteine protease enzyme that plays a crucial role in bone remodeling and the degradation of bone matrix proteins. It is primarily produced and secreted by osteoclasts, which are specialized cells responsible for breaking down and resorbing bone tissue during the bone remodeling process. Cathepsin K is a key enzyme involved in the resorption of bone, and its activity is essential for maintaining bone health.

The "calcitonin receptor (CTR)" is a G protein-coupled receptor (GPCR) found on the surface of various cells, including osteoclasts, which are specialized cells involved in bone remodeling. The primary function of the calcitonin receptor is to bind to calcitonin, a hormone produced by the thyroid gland, and mediate its effects in the body.

Calcitonin is released in response to high levels of calcium in the bloodstream, and its main role is to help regulate calcium homeostasis by lowering blood calcium levels. It does this by acting on its receptor, CTR, which is primarily found in bone tissue.

"Matrix metalloproteinase 9 (MMP-9)", also known as gelatinase B, is an enzyme belonging to a family of enzymes called matrix metalloproteinases (MMPs). MMPs are a group of proteolytic enzymes that play a critical role in the remodeling and degradation of the extracellular matrix, which is the non-cellular component of tissues that provides structural support and regulates various cellular processes. MMP-9, in particular, is involved in breaking down collagen and other components of the extracellular matrix.

"Carbonic anhydrase II (CAII)" is an enzyme that plays a critical role in the regulation of acid-base balance in the body, particularly in the transport of carbon dioxide and bicarbonate ions between tissues and the bloodstream. CAII belongs to a family of enzymes known as carbonic anhydrases, which catalyze the reversible conversion of carbon dioxide (CO₂) and water (H₂O) into bicarbonate ions (HCO³⁻) and protons (H⁺). This reaction is crucial for various physiological processes, including respiration, pH regulation, and ion transport.

An "arthritogenic virus vector" refers to a virus or vector organism that is capable of causing or contributing to the development of arthritis. Arthritis is a general term for inflammation of the joints, and certain viruses and vector organisms have been associated with the development of arthritic symptoms in some individuals. These viruses or vectors can trigger an autoimmune response or directly infect joint tissues, leading to joint inflammation and arthritis. However, when attenuated or rendered inactive, it still may serve as a vector to transport other material, in particular nucleic acids, to the joint.

Some examples of arthritogenic viruses and their associated vectors include: chikungunya virus (CHIKV), o'nyong-nyong virus (ONNV), Ross River virus (RRV), Barmah Forest virus (BFV), Mayaro virus (MAYV), Sindbis virus (SINV) and Semliki Forest virus (SFV).

"Intra-articular hyaluronic acid (IAHA)" is a medical treatment involving the injection of hyaluronic acid directly into a joint. This procedure is commonly used to manage symptoms and provide relief for individuals with osteoarthritis, a degenerative joint condition characterized by the breakdown of cartilage and inflammation within the joint.

Key points about intra-articular hyaluronic acid (IAHA) include:
- Hyaluronic Acid (HA): Hyaluronic acid is a naturally occurring substance found in the synovial fluid of joints, where it helps lubricate and cushion the joint. In osteoarthritis, the concentration and quality of HA in the joint can decrease, contributing to pain and reduced joint function.
- Procedure: During the IAHA procedure, a healthcare provider uses a sterile needle and syringe to inject hyaluronic acid directly into the affected joint. The procedure is typically performed in an outpatient setting and does not require general anesthesia.
- Frequency: The number of injections and the injection schedule can vary depending on the specific product used and the patient's response. In some cases, a single injection may be sufficient, while in others, a series of injections over several weeks may be recommended.
- Pain Relief: IAHA injections may provide pain relief and improve joint function for some individuals with osteoarthritis, particularly in the knee joint. The exact mechanism of action is not fully understood, but it is thought to involve improved lubrication and anti-inflammatory effects.

"Liposomes" are microscopic, spherical vesicles composed of one or more lipid bilayers (phospholipid or lipid molecules) that enclose an aqueous core. These lipid bilayers are similar in structure to the cell membranes found in living organisms. Liposomes are commonly used in various scientific, medical, and pharmaceutical applications due to their unique properties and versatility.

The "OARSI score", or the Osteoarthritis Research Society International score, is a tool used in clinical research and practice to assess the severity and progression of osteoarthritis in joints, particularly in the knee. Osteoarthritis is a degenerative joint disease that involves the breakdown of cartilage and other joint tissues, leading to pain, stiffness, and reduced joint function.

The OARSI score is a system for grading and staging the severity of osteoarthritis based on various clinical and radiological criteria. It helps healthcare professionals and researchers evaluate the extent of joint damage and formulate treatment plans. The score takes into account factors such as joint pain, joint swelling, range of motion, and radiographic findings (e.g., joint space narrowing and bone changes) to assess the overall condition of the affected joint.

The higher OARSI score, the worse the severity of osteoarthritis.

It provides a standardized way to categorize and communicate the severity of osteoarthritis, which can be helpful in monitoring disease progression and guiding treatment decisions.

The term "IC50" as used herein stands for "half-maximal inhibitory concentration." It is a measure used in pharmacology and biochemistry to describe the potency of a substance, typically a drug or chemical compound, in inhibiting a specific biological or biochemical function. The IC50 value represents the concentration of the substance needed to inhibit a particular response or biological activity by 50%.

In practical terms, a lower IC50 value indicates that a substance is more potent because it can achieve the desired inhibition at a lower concentration, while a higher IC50 value indicates that a higher concentration of the substance is needed to achieve the same level of inhibition.

IC50 values are commonly used in drug development and toxicology studies to assess the effectiveness of potential drugs or compounds in inhibiting specific enzymes, receptors, or biological processes. Researchers use IC50 values to compare the relative potency of different compounds and to determine their therapeutic or toxicological potential.

A "RNA sponge" or "microRNA (miRNA) sponge", also known as a "miRNA decoy" is defined herein as an artificial RNA construct designed to sequester and inhibit the activity of specific miRNAs within cells. A miRNA sponge is typically composed of multiple tandem repeats of miRNA-binding sites within a non-coding RNA scaffold. These binding sites are complementary to the target miRNA sequence, effectively competing with endogenous mRNA targets for miRNA binding. By introducing high concentrations of the miRNA sponge into cells, the sponge out-competes endogenous mRNA targets for binding to the miRNA. This sequestration leads to reduced availability of the miRNA to regulate its natural targets, thereby modulating gene expression.

Osteoarthritis (OA): OA is the most common form of arthritis and is often referred to as "wear and tear" arthritis. It typically occurs as a result of the breakdown of cartilage, the protective tissue that cushions the ends of bones in the joints. This can lead to pain, stiffness, and reduced joint mobility, especially in weight-bearing joints like the knees, hips, and spine.

Rheumatoid Arthritis (RA): RA is an autoimmune disease in which the body's immune system mistakenly attacks the synovium (the lining of the membranes that surround the joints). This leads to inflammation, pain, and joint damage. RA can affect multiple joints, including the small joints in the hands and feet.

Psoriatic Arthritis: Psoriatic arthritis is a type of arthritis that often occurs in individuals with psoriasis, a skin condition. It can affect both the skin and the joints, leading to joint pain, swelling, and skin lesions. Psoriatic arthritis can affect any joint in the body and may be mild or severe.

Ankylosing Spondylitis: This is a chronic inflammatory arthritis that primarily affects the spine and the sacroiliac joints (the joints between the spine and the pelvis). It can lead to pain, stiffness, and reduced mobility in the spine, and in severe cases, it can cause fusion of the vertebrae.

Gout: Gout is a type of arthritis caused by the buildup of uric acid crystals in the joints. It often affects the big toe but can also affect other joints. Gout attacks are known for their sudden and severe pain, redness, and swelling in the affected joint.

Juvenile Arthritis: Juvenile arthritis encompasses several types of arthritis that affect children and teenagers. The most common form is juvenile idiopathic arthritis (JIA), and it can have different subtypes with varying symptoms and joint involvement.

Systemic Lupus Erythematosus (SLE): While primarily an autoimmune disease that affects multiple organs and systems, SLE can also involve joint inflammation. Joint pain and swelling are common symptoms in lupus patients.

Sjögren's Syndrome: Sjögren's syndrome is an autoimmune disorder that primarily affects the glands that produce saliva and tears. However, it can also cause joint pain and inflammation in some individuals.

Osteoporosis: Although not typically classified as an arthritic disease, osteoporosis is a condition that weakens bones, making them more susceptible to fractures. It can lead to joint pain and decreased mobility as a result of fractures.

### Sequences of this disclosure

The sequence of the disclosed antagomiRs is shown as an RNA sequence in SEQ ID NO:2 and as a PNA sequence in SEQ ID NO:4. Compared to the sequences of SEQ ID NOs: 2 and 4 covering the entire sequence of the miR-574-5p (SEQ ID NO: 1), the sequences of SEQ ID NOs: 3 and 5 are five residues shorter, but still inhibit sufficiently miR-574-5p.
SEQ ID NO: 1 - miR-574-5p - 5 -tga gtg tgt gtg tgt gag tgt gt-3'
   (*respectively 5 -uga gug ugu gug ugu gag ugu gu-3' as RNA-sequence)
SEQ ID NO: 2 - RNA-antagomiR 1 - 5 -aca cac tca cac aca cac act ca-3'
   (*respectively 5 - aca cac uca cac aca cac acu ca -3' as RNA-sequence)
SEQ ID NO: 3 - RNA-antagomiR 2 - 5 -ctc aca cac aca cac tca-3'
SEQ ID NO: 4 - Peptide Nucleic Acid (PNA) antagomiR 1 - (5')-aca cac tca cac aca cac act ca-(3') (*with or without a modified lysine at 3'-terminus)
SEQ ID NO: 5 - Peptide Nucleic Acid (PNA) antagomiR 2 - (5')-ctc aca cac aca cac tca-(3') (*with or without a modified lysine at 3'-terminus)
SEQ ID NO: 6 - CPP 1 - GRKKRWFRRRRMKWKK
SEQ ID NO: 7 - penetratin ("P16" or "pAntp₄₃₋₆₈") - RQIKIWFQNRRMKWKK
SEQ ID NO: 8 - transactivator of transcription ("TAT" or "pTat₄₈₋₆₀") - YGRKKRRQRRR
   (*these are the amino acids 48-60 of the natural sequence of the HIV tat protein)
SEQ ID NO: 9 - model amphiphilic peptide (MAP) - KLALKLALKALKAALKLA
SEQ ID NO: 10 - polyarginines (including R3, R4, R5, R6, R7, R8, R9, R10, R11 and R12) - Argⁿ wherein n is selected from 3, 4, 5, 6, 7, 8, 9, 10, 11, 12, etc.
SEQ ID NO: 11 - pVEC - LLIILRRRIRKQAHAHSK
SEQ ID NO: 12 - transportan - GWTLNSAGYLLGKINLKALAALAKKIL
SEQ ID NO: 13 - Peptide Nucleic Acid (PNA) negative control ("ScrC")- (5)-gct att acc tta acc cag-(3')
   (*with or without a modified lysine at 3'-terminus)
SEQ ID NO: 14 - synthetic ath (*Arabidopsis thaliana*) - miR-159a - 5 -ttt gga ttg aag gga get cta-3')
   (*respectively 5 - uuu gga uug aag gga gcu cua-3' as RNA-sequence)
SEQ ID NO: 15 - synthetic cel (*Caenorhabditis elegans*) - miR-39-3p - 5'-tca ccg ggt gta aat cag ctt g-3'
   (*respectively 5 -uca ccg ggu gua aau cag cuu g -3' as RNA-sequence)
SEQ ID NO: 16 - NOXP20 fwd - 5'-ggc aaa tct ctg ctg tcg tc-3',
SEQ ID NO: 17 - NOXP20 rev - 5'-cct get ttt cct tga ctg c-3',
SEQ ID NO: 18 - GAPDH fwd - 5'-tga gaa cgg gaa get tgt ca-3',
SEQ ID NO: 19 - GAPDH rev - 5'-atc gcc cca ctt gat ttt gg-3',
SEQ ID NO: 20 - RT primer - 5'-gca tag acc tga atg tcg cta acg gtg tgg tag gcg aga cat ttt ttt ttt t-3'
SEQ ID NO: 21 - hsa-miR-574-5p fwd - 5'-gca gtg agt gtg tgt gtg a-3',
SEQ ID NO: 22 - hsa-miR-574-5p rev - 5'-gca tag acc tga atg tcg cta ac-3',
SEQ ID NO: 23 - ath-miR-159a fwd - 5'-gca gtt tgg att gaa ggg age t-3',
SEQ ID NO: 24 - ath-miR-159a rev - 5'-gca tag acc tga atg tcg cta ac-3',
SEQ ID NO: 25- polyarginine R4 - RRRR
SEQ ID NO: 26- polyarginine R5 - RRRRR
SEQ ID NO: 27- polyarginine R6 - RRRRRR
SEQ ID NO: 28- polyarginine R7 - RRRRRRR
SEQ ID NO: 29- polyarginine R8 - RRRRRRRR
SEQ ID NO: 30- polyarginine R9 - RRRRRRRRR
SEQ ID NO: 31- polyarginine R10 - RRRRRRRRRR
SEQ ID NO: 32 - polyarginine R11 - RRRRRRRRRRR
SEQ ID NO: 33 - polyarginine R12 - RRRRRRRRRRRR
SEQ ID NO: 34 - tartrate-resistant acid phosphatase (TRAP) -
SEQ ID NO: 35 - cathepsin K (cath K) -
SEQ ID NO: 36 - calcitonin receptor (CTR) 
MRFTFTSRCLALFLLLNHPTPILPAFSNQTYPTIEPKPFLYVVGRKKMMDAQYKCYDRMQ

QLPAYQGEGPYCNRTWDGWLCWDDTPAGVLSYQFCPDYFPDFDPSEKVTKYCDEKGVWFK

HPENNRTWSNYTMCNAFTPEKLKNAYVLYYLAIVGHSLSIFTLVISLGIFVFFRSLGCQR

VTLHKNMFLTYILNSMIIIIHLVEVVPNGELVRRDPVSCKILHFFHQYMMACNYFWMLCE

GIYLHTLIVVAVFTEKQRLRWYYLLGWGFPLVPTTIHAITRAVYFNDNCWLSVETHLLYI

IHGPVMAALVVNFFFLLNIVRVLVTKMRETHEAESHMYLKAVKATMILVPLLGIQFVVFP

WRPSNKMLGKIYDYVMHSLIHFQGFFVATIYCFCNNEVQTTVKRQWAQFKIQWNQRWGRR

PSNRSARAAAAAAEAGDIPIYICHQELRNEPANNQGEESAEIIPLNIIEQESSA
SEQ ID NO: 37 - matrix metalloproteinase 9 (MMP9) -
SEQ ID NO: 38 - carbonic anhydrase II (CAII) -
SEQ ID NO: 39 - macrophage colony-stimulating factor 1 (M-CSF or CSF-1) 
MTAPGAAGRCPPTTWLGSLLLLVCLLASRSITEEVSEYCSHMIGSGHLQSLQRLIDSQME

TSCQITFEFVDQEQLKDPVCYLKKAFLLVQDIMEDTMRFRDNTPNAIAIVQLQELSLRLK

SCFTKDYEEHDKACVRTFYETPLQLLEKVKNVFNETKNLLDKDWNIFSKNCNNSFAECSS

QDVVTKPDCNCLYPKAIPSSDPASVSPHQPLAPSMAPVAGLTWEDSEGTEGSSLLPGEQP

LHTVDPGSAKQRPPRSTCQSFEPPETPVVKDSTIGGSPQPRPSVGAFNPGMEDILDSAMG

TNWVPEEASGEASEIPVPQGTELSPSRPGGGSMQTEPARPSNFLSASSPLPASAKGQQPA

DVTGTALPRVGPVRPTGQDWNHTPQKTDHPSALLRDPPEPGSPRISSLRPQGLSNPSTLS

AQPQLSRSHSSGSVLPLGELEGRRSTRDRRSPAEPEGGPASEGAARPLPRFNSVPLTDTG

HERQSEGSFSPQLQESVFHLLVPSVILVLLAVGGLLFYRWRRRSHQEPQRADSPLEQPEG

SPLTQDDRQVELPV
SEQ ID NO: 40 - receptor activator of nuclear factor kappa-B ligand (RANKL) - 
MRRASRDYTKYLRGSEEMGGGPGAPHEGPLHAPPPPAPHQPPAASRSMFVALLGLGLGQV

VCSVALFFYFRAQMDPNRISEDGTHCIYRILRLHENADFQDTTLESQDTKLIPDSCRRIK

QAFQGAVQKELQHIVGSQHIRAEKAMVDGSWLDLAKRSKLEAQPFAHLTINATDIPSGSH
 
KVSLSSWYHDRGWAKISNMTFSNGKLIVNQDGFYYLYANICFRHHETSGDLATEYLQLMV

YVTKTSIKIPSSHTLMKGGSTKYWSGNSEFHFYSINVGGFFKLRSGEEISIEVSNPSLLD

PDQDATYFGAFKVRDID
SEQ ID NO: 41 - CPP with W/M-mutation: GRK KRW FRR RRM KMK K
SEQ ID NO: 42 - further truncated antagomiR: 5'-ctc aca cac aca cac-3'
   (*with or without a modified lysine at 3'-terminus)

In some embodiments the CPP-PNA-construct acting as inhibitor may be represented as (from N-terminus to C-terminus): GRKKRWFRRRRMKWKK-(AEEA-linker)-ctc aca cac aca cac tca-(K-CONH₂). The CPP-moiety in this conjugate corresponds to SEQ ID No: 6, the nucleic acid moiety in this conjugate corresponds to PNA with SEQ ID No: 5, the conjugate further comprises a modified lysine at the 3' terminus, which was modified with a amid-group (-CONH₂) instead of carboxyl-group (-COOH), cf. also figure 1. This inhibitor was used in the experiments when the terminology "miR-574-5p-inhibitor" was used.

Generally, the CPP can be selected from SEQ ID No: 6 - 12, or SEQ ID No 25 - 33, or SEQ ID NO: 41, in one embodiment SEQ ID No: 6, the expression "linker" may be in one embodiment a PEG-linker comprising one ethylene glycol-unit, in further embodiments an AEEA-linker, or in yet other embodiments any of the linkers described hereinunder. Coupled to the linker is an antagomir according to SEQ ID No: 2-5, or 42. In other embodiments the PNA-antagomir may be SEQ ID No: 5. At the end the molecule may comprise a modified lysin with a amid-group (-CONH₂) instead of carboxyl-group (-COOH).

Some CPP-antagomiR conjugates according to the disclosure are:
- GRKKRWFRRRRMKWKK-(PEG-linker)-aca cac tca cac aca cac act ca-(K-CONH₂)
- GRKKRWFRRRRMKWKK-(PEG-linker)-ctc aca cac aca cac tca-(K-CONH₂)
- GRKKRWFRRRRMKWKK-(PEG-linker)-ctc aca cac aca cac-(K-CONH₂)
- GRKKRWFRRRRMKWKK-(AEEA linker)-aca cac tca cac aca cac act ca-(K-CONH₂)
- GRKKRWFRRRRMKWKK-(AEEA linker)-ctc aca cac aca cac tca-(K-CONH₂)
- GRKKRWFRRRRMKWKK-(AEEA linker)-ctc aca cac aca cac-(K-CONH₂)
- GRKKRWFRRRRMKMKK-(PEG-linker)-aca cac tca cac aca cac act ca-(K-CONH₂)
- GRKKRWFRRRRMKMKK-(PEG-linker)-ctc aca cac aca cac tca-(K-CONH₂)
- GRKKRWFRRRRMKMKK-(PEG-linker)-ctc aca cac aca cac-(K-CONH₂)
- GRKKRWFRRRRMKMKK-(AEEA linker)-aca cac tca cac aca cac act ca-(K-CONH₂)
- GRKKRWFRRRRMKMKK-(AEEA linker)-ctc aca cac aca cac tca-(K-CONH₂)
- GRKKRWFRRRRMKMKK-(AEEA linker)-ctc aca cac aca cac-(K-CONH₂)
- GRKKRWFRRRRMKWKK-(PEG-linker)-aca cac tca cac aca cac act ca
- GRKKRWFRRRRMKWKK-(PEG-linker)-ctc aca cac aca cac tca
- GRKKRWFRRRRMKWKK-(PEG-linker)-ctc aca cac aca cac
- GRKKRWFRRRRMKWKK-(AEEA linker)-aca cac tca cac aca cac act ca
- GRKKRWFRRRRMKWKK-(AEEA linker)-ctc aca cac aca cac tca
- GRKKRWFRRRRMKWKK-(AEEA linker)-ctc aca cac aca cac
- GRKKRWFRRRRMK**M**KK-(PEG-linker)-aca cac tca cac aca cac act ca
- GRKKRWFRRRRMK**M**KK-(PEG-linker)-ctc aca cac aca cac tca
- GRKKRWFRRRRMK**M**KK-(PEG-linker)-ctc aca cac aca cac
- GRKKRWFRRRRMK**M**KK-(AEEA linker)-aca cac tca cac aca cac act ca
- GRKKRWFRRRRMK**M**KK-(AEEA linker)-ctc aca cac aca cac tca
- GRKKRWFRRRRMK**M**KK-(AEEA linker)-ctc aca cac aca cac
as well as any functional derivative thereof.

In further embodiments the disclosure pertains to the following further configurations for the inhibitor comprised by the disclosure:

| **CPP** | **Linker** | **Nucleic acid part** | **End-group** |
|---|---|---|---|
| Selected from | Selected from PEG (from 1 to 175 units), or AEEA-linker, and/or any other linker moiety disclosed herein | Backbone selected from RNA, DNA, LNA, PNA, BNA, PMO; | selected from none, amino acid with carboxyl-group (-COOH) and modified amino acid with amid-group (-CONH₂), such as a modified lysin with a amid-group |
| SEQ ID No: 6 -12, or 25 - 33 and/or SEQ ID NO: 41 | | | |
| | | and | |
| | | Sequence selected from SEQ ID No: 2-5, and/or SEQ ID NO: 42 | |

As a negative control, a PNA with a sequence not complementary to miR-574-5p was tested which was also bound to CPP via an AEEA linker. The negative control CPP-PNA construct (also called "ScrC" hereinunder) which was used in the experiments can be represented as follows (from N-terminus to C-terminus): GRKKRWFRRRRMKWKK-(AEEA-linker)-gct att acc tta acc cag-(K-CONH₂). The CPP-moiety in this conjugate corresponds to SEQ ID No: 6, the nucleic acid moiety in this conjugate corresponds to PNA with SEQ ID No: 13, the conjugate further comprises a modified lysine at the 3' terminus, which was modified with a amid-group (-CONH₂) instead of carboxyl-group (-COOH), cf. also figure 1. This negative control was used in the experiments wherever the term "miR-574-5p negative control" or "ScrC" was used.

Another negative control used in the experiments consists of the CPP of SEQ ID No: 6 only, in those cases the term "CPP" or "CPP-control" was used.

### EXAMPLES

### Cell culture

Cells were cultured in DMEM supplemented with 10% (v/v) fetal bovine serum (FBS), 100 U/mL penicillin, 100 µg/mL streptomycin, and 1 mM sodium pyruvate (all Gibco Life Technologies, Thermo Fisher Scientific, Waltham, USA). A humidified atmosphere at 37°C and 5% CO₂ was maintained throughout during propagation.

A549 human adenocarcinoma and Hep G2 human hepatoma cells were purchased from ATCC (Manassas, USA). Cell lines were subcultured twice a week. CD14⁺ monocytes were isolated freshly for each experiment from buffy coats obtained from anonymous healthy donors through the DRK*-Blutspendedienst-Blutspendezentrum* (Frankfurt a.M., GER).

Cells were seeded at the indicated concentrations for experimental purposes.

### CD14⁺ monocyte isolation and osteoclast differentiation

PBMCs were isolated from buffy coats by Ficoll-Paque Plus (GE Healthcare, Chicago, USA) density gradient separation.

Briefly, 15 ml of the buffy coat was mixed 1:1 with 1 x PBS and underlaid with 7 ml Ficoll. The PBMCs underwent separation by centrifugation for 20 min at 800 x g in a swing-out rotor. The PBMC layer was carefully harvested with a micropipette, transferred to a fresh tube, and washed twice with 1x PBS (10 min at 500 x g). The PBMC pellet obtained was then resuspended in ice-cold MACS buffer (1 mM EDTA and 2% FBS in 1x PBS). The PBMCs were then passed through a 30 µm pre-separation filter and washed once with ice-cold MACS buffer (10 min at 500 x g). The pellet was resuspended in 1 ml MACS and CD14+ cells were labeled with 100 µl CD14 MicroBeads (Miltenyi Biotec, Bergisch Gladbach, GER) for 30 min at 4°C. After an additional MACS buffer wash step, PBMCs resuspended in 6 ml MACS were applied to a pre-equilibrated LS MACS column mounted in MediMACS Separator (both Miltenyi Biotec, Bergisch Gladbach, GER). The column was washed with two column volumes of MACS buffer. CD14+ cells were eluted in 6 ml MACS buffer by removing the column from the MACS separator. The CD14+ cells were washed once in 1x PBS, and the cell concentration was determined. Finally, CD14+ cells were resuspended at a concentration of 5×10⁵ cells/ml in a culture medium supplemented with 25 ng/ml M-CSF (PeproTech Inc., East Windsor, USA). After three days of cultivation, the medium was changed to a fresh culture medium supplemented with 10ng/ml M-CSF and 5ng/ml RANK-L (R&D Systems Inc., Minneapolis, USA). This process was repeated every three days until 12 days of continuous culture.

### miR-574-5p inhibitor

miR-574-5p inhibitor (comprising SEQ ID NO: 5 and SEQ ID NO: 6) and miR-574-5p negative control (comprising SEQ ID NO: 6 and SEQ ID NO: 13) were custom ordered from biomers.net (Ulm, GER). The peptide-peptide nucleic acid hybrid molecules were continuously constructed by standard methods of solid phase synthesis. The inhibitors were stored lyophilized at -20°C. For experimental procedures, molecules were resuspended in sterile 1x PBS and stored at -20°C until further use.

### FISH

1.5×10⁴ A549 cells were seeded into 8-well cell culture slides (spl life science, Naechon-Myeon, KOR) and cultured for 24h. The next day, the cells were stimulated with 5µM miR-574-5p inhibitor or miR-574-5p negative control. The culture medium was discarded, and the cells were washed once with PBS after 4 hours of incubation. Cells were fixed in formaldehyde for 10 min at room temperature (RT) (4% in 1x PBS, Carl Roth, Karlsruhe, GER) followed by Triton X-100 permeabilization (0.5% in 1x PBS, 10 min at RT, Carl Roth, Karlsruhe, GER). After three PBS-T (0.01% Tween20 in 1x PBS) washes, samples were transferred to a hybridization chamber and incubated with double digoxigenin-labeled anti-miR-574-5p probe (Qiagen, Hilden, GER, 100 nM in 1x Qiagen miRNA ISH buffer). Samples were hybridized at 54°C for 1 h. Unbound probe was washed out twice with 54°C warm 2x SSC buffer (Invitrogen Karlsruhe, GER). Blocking was performed with 2% BSA (in PBS, 30 min at RT). The detection of the probe was performed overnight at 4°C with rabbit anti-DIG antibodies (Thermo Fisher Scientific, Waltham, USA, 1 ng/ml final concentration, diluted in blocking buffer). Three PBS-T washes removed unbound antibodies. Anti-DIG antibodies were detected using donkey anti-rabbit AlexaFluor 594 conjugated antibodies (abcam, Cambridge, UK, ab150080, 5 ng/ml final, diluted in blocking buffer). After incubation for 1 hour at RT, the samples were washed three times with PBS-T. Cell nuclei were counterstained with 4',6-diamidino-2-phenylindole (DAPI, Sigma-Aldrich, Darmstadt, GER, 1 µg/ml, 10 min at RT). The samples were washed once with water and mounted with Mowiol (Sigma-Aldrich, Darmstadt, GER).

### Image acquisition and miRNA signal quantification

Stained cells were visualized using a Leica DMi8 confocal microscope with a 20x water immersion objective. Analysis of micrographs was performed using a custom imaged macro. Regions of interest were defined by the nuclear DAPI signal. Single cell measurements of the average fluorescent miRNA signal were performed. For each replicate condition, at least 800 single cell measurements were averaged. The mean of the treated group was divided by the mean of the untreated group to determine the relative miRNA signal.

### Immunofluorescence staining

5×10⁵ CD14⁺ cells were seeded in 24-well plates with 12 mm glass coverslips and differentiation was initiated as described above. Coverslips were removed after 3, 6, 9 or 12 days and fixed with formaldehyde (10 min at RT, 4% in 1x PBS). The samples were then permeabilized with Triton X-100 (0.5% in 1x PBS) for 10 min at RT. Samples were blocked with 3% BSA (in 1x PBS) for 30 min at RT. The samples were then incubated with their respective primary and secondary antibodies (Tab. 1) for 1 hour at RT. At the end of each step, the samples were washed three times with PBS-T. Finally, nuclei and actin cytoskeletons were counterstained with DAPI (1 µg/ml) and phalloidin-iFluor 647 (1:1000; Abcam, Cambridge, UK) for 10 min. Coverslips were rinsed in distilled water and mounted with Mowiol. Micrographs were taken using a Leica DMi8 confocal microscope with a 63x water immersion objective.

### RNA extraction

Intracellular RNA was extracted with TRIzol reagent (Thermo Fisher Scientific, Waltham, USA) and digested with Turbo DNase (Thermo Fisher Scientific, Waltham, USA) according to the manufacturer's instructions. Samples were spiked with 0.01 pmol synthetic ath (*Arabidopsis thaliana*)-miR-159a (5'-ttt gga ttg aag gga get cta-3') (SEQ ID NO. 14) and 1 pmol cel (*Caenorhabditis* e/egans)-miR-39-3p (5'-tca ccg ggt gta aat cag ctt g-3', (SEQ ID NO. 15) both Sigma Aldrich, Darmstadt, GER). Spiked in cel-miR-39-3p was used as a co precipitant to increase miRNA recovery efficiency and ath-miR-159a was used as an external standard for normalization of miR-574-5p expression.

### mRNA quantitative reverse transcription chain reaction (RT-qPCR)

cDNA synthesis for mRNA detection was performed using the High-Capacity cDNA Reverse Transcription Kit (Applied Biosystems, Thermo Fisher Scientific, Waltham, USA) according to the manufacturer's instructions.

QPCR was performed with PerfeCTa FastMix II (Quantabio, Beverly, USA) according to the manufacturer's instructions in a StepOne RT-PCR system (Applied Biosystems, Thermo Fisher Scientific, Waltham, USA). Transcript-specific cDNA amplification was performed using the following primers: NOXP20 (SEQ ID NO. 16), (SEQ ID NO. 17), GAPDH (SEQ ID NO. 18), (SEQ ID NO. 19), all primers Sigma Aldrich, Darmstadt, GER).

### miRNA RT-qPCR

The cDNA used to detect miRNA was prepared. Briefly, a 20 µl reaction was set up containing: 2 µl extracted total RNA, 0.1 U/µl poly(A) polymerase, 5 U/µl MulV reverse transcriptase, 0.55 mM ATP, 0. 5 mM dNTP and 0.5 µM RT primer (SEQ ID NO. 20) in 1x poly(A) buffer (all New England Biolabs, Ipswich, USA). Reverse transcription was then performed at 42°C for 1 hour.

RT-qPCR was performed with the same materials and instrumentation as described above. MiRNA-specific amplification was achieved using the following primers: hsa-miR-574-5p (SEQ ID NO. 21) and (SEQ ID NO. 22) and ath-miR-159a (SEQ ID NO. 23) (SEQ ID NO. 24), all primers Sigma Aldrich, Darmstadt, GER).

### ELISA

Quantification of serum CTX-II was performed using the Serum Pre-Clinical CartiLaps (CTX-II) ELISA Kit (Immunediagnostic Systems, Boldon, UK). Serum of mice at the pre-OP phase and after sacrifice was analyzed according to the manufacturer's instructions. Samples were diluted 1:2 in Ca!0 buffer and assayed in duplicate. Due to the limited availability of serum in the pre-OP phase, 3 samples were only assayed as unikats.

### TRAP staining

TRAP staining was performed using an acid phosphatase, leukocyte staining kit (Sigma-Aldrich, Darmstadt, GER). Fixative- and staining solution were prepared according to the manufacturer's instructions. The cell culture medium was discarded, and the wells were washed once with 1x PBS. Cells were then fixed with 50 µl fixative for 1 minute. The wells were rinsed twice with distilled water. Freshly prepared 37°C warm staining solution was added and incubated at 37°C for 15 min or until sufficient staining developed. Staining solution was aspirated, and each well was washed twice with distilled water. Finally, the stained cells were air dried at RT overnight. TRAP-positive multinucleated cells (>3 nuclei) were considered mature OCs and were manually quantified under a light microscope at 10x magnification. Relative OC counts were determined by normalizing the mean of the treated groups to the mean of the untreated group.

### MTT viability assay

CD14⁺ monocytes were isolated and seeded as described above. 24 hours after treatment, 20 µl MTT solution (5 mg/ml in 1x PBS, Carl Roth, Karlsruhe, GER) was added per well and allowed to metabolize for 4 hours. The culture medium was discarded, and the cells were lysed by adding 100 µl DMSO. The amount of metabolized MTT was quantified by determining the absorbance at a wavelength of 570 nm. The absorbance at 630 nm was also determined and used to normalize. The mean value of each treatment group was normalized to the mean value of the untreated group to calculate relative viability.

### Animal Husbandry

Female C57BI/6J mice, 8 weeks old with a mean weight of 20 g (range 17-22 g), were purchased from Scandbur, Denmark and housed 6 mice per cage in dedicated pathogen-free facilities at Karolinska University Hospital. Animals were acclimatized for 7 days prior to any intervention. All experiments were approved by the Ethical Committee of Stockholm North (Dnr 16033-2021). A 12-hour light/dark cycle was maintained, and animals had free access to water and standard rodent chow. The mice were checked daily, and their weight was monitored weekly throughout the experiment to ensure their well-being.

### ACLT surgery

OA was induced by surgically destabilizing the left knee joint by ACLT (anterior cruciate ligament tear). Briefly, a skin incision was made, and the joint space was localized and accessed with a 90 degree "hooked" 23G needle, after which the ACLT was carefully performed with an 11-blade scalpel without further exposure of the joint. Destabilization was ensured by controlling the rotational capacity before and after transection. In sham control mice, a skin incision was made and immediately closed with sutures.

The surgery was performed under isoflurane anesthesia, and postoperative pain relief was provided by subcutaneous administration of buprenorphine (0.3 mg/ml) for the first 48 hours. Mice were allowed to move freely in their cages after surgery and were monitored daily. Animals were randomized into treatment groups prior to surgery. ACLT-treated mice received two intra-articular injections (10 µl containing 4 nmol miR-574-5p inhibitor or control) under isoflurane anesthesia, one at the time of surgery and one after 4 weeks.

Urine samples were collected preoperatively, at day 3, 1 week, 2 weeks, 4 weeks, 6 weeks, and 8 weeks. Blood samples were drawn from the femoral vein 1 week before surgery and 8 weeks post-surgery at the point of sacrifice. Animals were sacrificed 8 weeks after surgery and joints were processed for histopathologic examination.

### Safranin-O staining

Knee joints were fixed in 4% phosphate-buffered formaldehyde for 24 hours, decalcified in EDTA solution, dehydrated, and embedded in paraffin. Frontal 6 µm sections were cut and serial sections were collected. For histologic evaluation, central joint sections were stained with Safranin-O/Fast Green. Briefly, slides were deparaffinized in three changes of xylene and hydrated through 100%, 95%, and 70% ethanol. Sections were thoroughly air-dried and stained with Weigert's iron hematoxylin (Sigma-Aldrich, Darmstadt, GER) for 7 min. After washing under running tap water for 10 min, sections were immersed in 0.08% Fast Green (Sigma-Aldrich, Darmstadt, GER) for 3 min. Sections were dipped once in 1% acetic acid and then immersed in 0.1% Safranin O (Sigma-Aldrich, Darmstadt, GER) for 5 min. The sections were then washed in two changes of distilled water and air-dried. Finally, the slides were cleared in one change of xylene and covered by a coverslip of Histokitt II (Carl Roth, Karlsruhe, GER).

### Semi-quantitative histopathology

Joint sections were divided into four quadrants along the femoral-tibial and medial-lateral axes. Each quadrant was scored semi-quantitatively according to the OARSI Histopathology Initiative recommendations for mice. Four additional scores were introduced to reflect overall joint pathology with respect to osteophyte formation, meniscal swelling, synovial membrane inflammation, and ligament hypertrophy and chondrogenesis. Each score ranges from 0 (normal morphology) to 1 (pathologic phenotype) and 2 (severe pathologic phenotype). Sections were evaluated by two independent, blinded observers.

### Knee-ISH

Paraffin-embedded frontal knee sections were deparaffinized in 3 changes of xylene (Carl Roth, Karlsruhe, GER) and rehydrated through graded ethanol baths (100%, 95%, and 70%). This was followed by 3 washes in distilled water. Antigen retrieval was performed with 3 µg/ml Proteinase K (Qiagen, Hilden, GER) at 37°C for 10 min. After two washes in 1x PBS, prehybridization was performed at 50°C for 20 min (in 1x ISH buffer, Qiagen, Hilden, GER). Subsequently, hybridization was performed at 55°C for 1 hour with 100 nM locked nucleic acid (LNA) miRNA probe (in 1x ISH-buffer, double fluorescein labeled, Qiagen, Hilden, Germany) directed against miR-574-5p. Unbound probe was washed out with 50°C pre-warmed saline sodium citrate buffer (SSC, Invitrogen, Karlsruhe, GER) according to the following scheme: Once with 5x SSC, twice with 1x SSC and twice in 0.2x SSC, each for 5 min. Tissue sections were then blocked in 1x PBS containing 0.1% Tween20, 2% sheep serum (Jackson Immunoresearch, Ely, UK) and 1% BSA for 15 min. Probe detection was then performed with anti-fluorescein alkaline phosphatase labeled antibody (11 426 338 910, used 1:500 in blocking buffer, Roche, Basel, CHE). After three washes with 1x PBS containing 0.1% Tween20, alkaline phosphatase activity was detected for 2 hours at 30°C using nitro blue tetrazolium/5-bromo-4-chloro-3-indolyl phosphate (NBT-BCIP, Roche, Basel, CHE) as a substrate. The reaction was stopped by washing in KTBT buffer (50 mM Tris-HCL, 150 mM NaCl, 10 mM KCI) twice for 5 minutes each. The knees were then counterstained with nuclear fast red (Vector Laboratories, Burlingame, USA) for 1 min. Excess dye was washed out under running tap water for 10 min. Sections were dehydrated through graded ethanol baths. Finally, the tissue was cleared in xylene and coverslipped using HistoKit II.

### Statistics

Each experiment was performed with at least three biological replicates (see corresponding figure for details). For cell culture experiments, each biological replicate was the mean of at least 3 technical replicates. Significance of relative changes was tested using paired one-way analysis of variance (ANOVA). The significance of differences in ordinal data was tested using the Mann-Whitney test. Significance was calculated using Prism 9 (GraphPad Software, Boston, USA) and p values are indicated as follows: * p<0.05; ** p<0.01; *** p<0.001. $ indicates significance compared to unplotted untreated controls.

### MiR-574-5p-inhibitor efficiently reduces miR-574-5p expression in A549 cells

First, the ability of the miR-574-5p inhibitor to cross the cell membrane and its effect on miR-574-5p expression was investigated in-vitro. Therefore, A549 cells were treated with the miR-574-5p inhibitor (comprising SEQ ID NO: 5 and SEQ ID NO: 6) or controls, i.e., the ScrC control (miR-574-5p negative control comprising SEQ ID NO: 6 and SEQ ID NO: 13) or the CPP part alone (SEQ ID NO. 6), and their effects on endogenous miR-574-5p levels was monitored using quantitative fluorescent in-situ hybridization (FISH) and quantitative reverse transcription chain reaction (RT-qPCR). To monitor the early effects of the inhibitor on miR-574-5p, A549 cells were incubated in the presence of the inhibitor for 4 hours to allow cellular uptake and binding to its miR-574-5p target. The cells were then fixed and non-masked miR-574-5p was detected by FISH (Fig. 2A). Freely available miR-574-5p (SEQ ID NO. 1) was analysed and quantified in a high-throughput manner. The average of at least 800 single cell measurements for each condition was normalized to an untreated control (Fig. 2B). After 4 hours of treatment, the FISH signal intensity of miR-574-5p inhibitor-treated cells decreased significantly by 25%, while the intensity of CPP- and ScrC-treated cells was not significantly affected. Further the knockdown efficiency by RT-qPCR was analysed. After 24 hours of treatment, a 90% reduction of endogenous miR-574-5p levels (SEQ ID NO. 1) was measured (Fig 2C). The knockdown effect was long lasting and persisted after 48 hours. The reduction in miR-574-5p levels was not associated with a reduction in the expression of the miR-574-5p host gene NOXP2018 (Fig. 2D). NOXP20 levels remained unchanged after a 24 hour of treatment with either inhibitor or their respective controls.

In conclusion, the miR-574-5p inhibitor (comprising SEQ ID NO: 5 and SEQ ID NO: 6) was able to cross the cell membrane of A549 cells and to cause a significant reduction of miR-574-5p expression within 3 hours. Moreover, its knockdown efficacy reached 90% after 24 hours without affecting host gene expression.

### MiR-574-5p inhibitor (comprising SEQ ID NO: 5 and SEQ ID NO: 6) significantly reduces osteoclast differentiation and PGE2-biosynthesis in-vitro

In the next step, we investigated whether the inhibition of miR-574-5p (SEQ ID NO. 1) reduces the formation of osteoclasts. For this purpose, CD14+ monocytes were isolated from peripheral blood mononuclear cells (PBMCs) of healthy donors and stimulated with recombinant human M-CSF (SEQ ID NO. 39) as well as RANKL (SEQ ID NO. 40) for 12 days(Fig. 3A). Cells were treated on day 3 of osteoclast differentiation with various concentrations (1 - 10 µM) of the inhibitor or its controls, the ScrC control (miR-574-5p negative control comprising SEQ ID NO: 6 and SEQ ID NO: 13), or the CPP part alone (SEQ ID NO. 6). On day 12, cells were fixed and stained for tartrate resistant phosphatase (TRAP) (SEQ ID NO. 34), and the number of osteoclasts was counted. We observed a significant dose-dependent de-crease (-80% at 10 µM treatment) in the number of osteoclasts upon application of the inhibitor (Fig. 3B/C) with an IC₅₀ value of 5 µM. To assess whether differences in time and stage of maturation influence the effect of the inhibitor, freshly isolated CD14+ monocytes were also treated with the compounds. Again, a dose-dependent reduction to -80% was observed upon treatment with the inhibitor.

Cell viability was determined using the tetrazolium reduction assay (Fig. 3D). This was done to exclude the possibility that the observed effect of the inhibitor was due to cytotoxicity. In this assay, 3-(4,5-dimethylthiazol-2-yl)-2,5-diphenyltetrazolium bromide (MTT) is reduced to insoluble formazan by the metabolic activity of living cells34. In macrophages, no effect on cell viability or cell metabolism was observed 24 hours after treatment with the miR-574-5p inhibitor and its controls (comprising SEQ ID NO: 6 and SEQ ID NO: 13; and CPP SEQ ID NO: 6 alone) (Fig. 3D). In CD14+ monocytes, the treatment with the different components resulted in a slight decrease in the average cell viability but cell viabilities, but cell viability did not fall below the 70% threshold. To further validate the cytotoxicity of the miR-574-5p inhibitor, also the MTT assay with Hep G2 cells was used. Again, at the tested concentrations of 1-20 µM, no negative effect on cell viability was observed for either the miR-574-5p inhibitor (comprising SEQ ID NO: 5 and SEQ ID NO: 6) or the CPP control (SEQ ID NO. 6). Interestingly, a dose-dependent cytotoxic effect was observed for ScrC (comprising SEQ ID NO: 6 and SEQ ID NO: 13), and a concentration of 10 µM resulted in a viability reduction below the 70% threshold.

To determine whether minimal variations in the sequence of the CPP fragment (SEQ ID NO. 6) or the antisense oligonucleotide fragment (SEQ ID NO. 5) affect the intrinsic effect of the inhibitor, we modified the sequences. Therefore, two modified inhibitors were analyzed: (1) a truncated miR-574-5p inhibitor which lacks the three most c-terminal PNA nucleotides as well as the c-terminal lysine (comprising SEQ ID NO. 6 and SEQ ID NO. 42), and (2) the full-length inhibitor with a W14M amino acid substitution in the CPP (comprising SEQ ID NO. 6 and SEQ ID NO. 41). The intrinsic effect on osteoclast formation was not affected by the sequence variations. However, both changes negatively affected cell viability at the macrophage stage compared to the unaltered miR-574-5p inhibitor.

We further investigated whether the inhibition of PGE2 formation by the miR-574-5p inhibitor (comprising SEQ ID NO: 5 and SEQ ID NO: 6) contributes to its inhibitory effect on osteoclast maturation. For this purpose, we performed osteoclastogenesis assays and treated either monocytes or macrophages with different concentrations (1-20 µM) of two inhibitors of cellular PGE2 formation: Celecoxib was chosen as an inhibitor of cyclooxygenase 2 (COX2). Compound III (CIII) was chosen as inhibitor of the microsomal PGE2 synthase 1 (mPGES1). In the case of celecoxib a concentration-dependent decrease in osteoclast numbers was observed. However, this dose-dependent response was accompanied by a dose-dependent decrease in cell viability. In contrast, CIII showed no significant cytotoxicity at the concentrations tested and was able to reduce the relative osteoclastogenesis in a dose-dependent manner. At 10 µM, CIII is able to reduce osteoclastogenesis to about 60-70%.

In conclusion, the miR-574-5p inhibitor was able to specifically inhibit osteoclast maturation in an in-vitro model of primary cell osteoclastogenesis without negatively affecting cell viability.

### MiR-574-5p inhibitor (comprising SEQ ID NO: 6 and SEQ ID NO: 6) shows in-vivo efficacy in an ACLT mouse model of OA

To test whether the miR-574-5p inhibitor (comprising SEQ ID NO: 5 and SEQ ID NO: 6) is able to improve joint pathology, we used the ACLT mouse model and injected the miR-574-5p inhibitor (4 pmol) into the affected joint twice (at the time of surgery and 4 weeks after surgery). The experiment was terminated after 8 weeks and joint pathology was evaluated (Fig. 5A). Frontal knee sections were taken and stained with Safranin O/Fast Green to assess the overall cartilage damage (Fig. 5b). The micrographs show a section of the medial knee joint with the tibial growth plate at the bottom (stained red), the sub-chondral bone above (stained green/blue), and the tibial surface cartilage at the top. Parts of the meniscus are visible in the joint space. The femoral articular surface cartilage and its subchondral bone are seen in the upper part of the micrograph.

Articular surface cartilage damage was then scored according to the OARSI guidelines for mice. Therefore, we further divided the knee sections into quadrants along the femoral-tibial and medial-lateral axes and scored each quadrant individually in a blinded fashion. The combined OARSI scores were then plotted and compared (Fig. 5c). Considering all four quadrants, the analysis showed that both CPP- and ScrC-treated knees exhibited a significantly worse phenotype compared to sham-operated knees, whereas the miR-574-5p inhibitor-treated knees did not differ significantly from the sham group. The sham group showed a combined OARSI score of 8 (median) compared to 16.5 and 15 of the CPP and ScrC groups, respectively. The miR-574-5p inhibitor-treated group had a median combined OARSI score of 11, with two-thirds of the treated mice exceeding the sham-treated baseline.

This difference between treatment and sham mice was more pronounced in the medial quadrants. The sham group showed a combined OARSI value of 1.5. CPP and ScrC were significantly different with values of 9 and 7, respectively. The miR-574-5p inhibitor group exhibited a reduced OARSI value of 5.5. It is noteworthy that three out of seven treated mice reached the sham treatment baseline value. The combined OARSI values in the lateral quadrants showed a similar trend to the medial quadrants. However, the median value was higher in the sham group (5.5 vs. 1.5). Only the CPP control group differed significantly from the sham and miR-574-5p inhibitor groups. Analysing the combined OARSI scores for tibia and femur showed no significant differences between sham and treated groups.

Other signs of chronic joint damage, such as osteophyte formation, ligamentous and synovial hyperplasia, were further analysed in terms of overall joint pathology (Fig. 4D). As expected, we observed an increase in osteophyte formation in operated versus sham-operated mice. However, there was no trend between the different treatment groups. An increase in synovitis was also observed in the operated mice. This was significantly higher in the ScrC-treated group than in the sham-operated group. In contrast, the treatment with the miR-574-5p inhibitor resulted in a significant reduction of synovitis in comparison to the treatment with ScrC. The same trend was observed when ligament hyperplasia was analysed. Compared to the sham-operated baseline, both control treatments showed a significant increase in ligament hyperplasia. When the operated mice were treated with the miR-574-5p inhibitor, this difference with the sham group was no longer significant.

Finally, we quantified serum levels of collagen type II C-terminal telopeptide (CTX-II) to monitor cartilage degeneration (Fig. 4E). CTX-II is a by-product of enzymatic cartilage degradation. Its levels correlate with cartilage damage. We showed that the level of CTX-II was significantly reduced by miR-574-5p inhibitor compared to the CPP or ScrC treated group. In particular, the serum level of CTX-II was lower in the miR-574-5p inhibitor treated mice compared to the same-operated mice.

In conclusion, our miR-574-5p inhibitor significantly reduced cartilage damage in an ACLT mouse model of OA and reduces OARSI scores in two-thirds of the mice below the baseline of the sham-operated group. This was mainly due to a reduction of cartilage damage in both medial quadrants. In addition, treatment with the miR-574-5p inhibitor showed a reduction in synovitis and ligament hyperplasia to levels which not significantly different from the sham-operated baseline.

## Claims

1. An inhibitor of miR-574-5p (SEQ ID NO: 1) for use in intra-articular treatment of a disease selected from the group consisting of osteoarthritis (OA), rheumatoid arthritis (RA), psoriatic arthritis, ankylosing spondylitis, gout, juvenile arthritis, systemic *lupus erythematosus* (SLE), Sjögren's syndrome and osteoporosis.

2. The inhibitor of miR-574-5p for use according to claim 1, wherein the intra-articular treatment is selected from an intra-articular injection into the joint or an arthroscopic lavage.

3. The inhibitor of miR-574-5p for use according to claim 1, wherein the inhibitor of miR-574-5p is a nucleic acid-based inhibitor.

4. The inhibitor of miR-574-5p for use according to claim 3, wherein the sequence of the nucleic acid-based inhibitor is at least 90%, at least 95%, at least 99% complementary to miR-574-5p (SEQ ID NO: 1) and, optionally, wherein the nucleic acid-based inhibitor is single-stranded.

5. The inhibitor of miR-574-5p for use according to claim 3, wherein the nucleic acid-based inhibitor comprises a nucleic acid-element selected from the group consisting of deoxyribonucleic acid (DNA), ribonucleic acid (RNA), peptide nucleic acid (PNA), locked nucleic acid (LNA), Morpholino-oligonucleotides, peptide-oligonucleotide hybrids (PONH), xeno-nucleic acid (XNA) and aptamers, as well as combinations thereof.

6. The inhibitor of miR-574-5p for use according to at least one of the preceding claims, wherein inhibitor is conjugated to a cell-penetrating peptide (CPP).

7. The inhibitor of miR-574-5p for use according to claim 6, wherein the cell penetrating peptide (CPP) is selected from the group consisting of a CPP according to SEQ ID NO: 6, penetratin (SEQ ID NO: 7), transactivator of transcription (TAT) (SEQ ID NO: 8), model amphiphatic peptide (MAP) (SEQ ID NO: 9), polyarginines (including R3, R4, R5, R6, R7, R8, R9, R10, R11 and R12) (SEQ ID NO: 10 and/or SEQ ID NO: 25-33), pVEC (SEQ ID NO: 11), and transportan (SEQ ID NO: 12), as well as functional derivatives, peptidomimetics and combinations thereof.

8. The inhibitor of miR-574-5p for use according to at least one of the preceding claims, wherein the inhibitor comprises PNA and is conjugated to the cell penetrating peptide (CPP) comprising SEQ ID NO: 6, optionally, via a linker.

9. The inhibitor of miR-574-5p for use according to claim 8, wherein the linker is selected from the group consisting of PEG-linkers, AEEA-linkers, glycine-serine linkers, polyalanine linkers, coiled-coil linkers, helical linkers, cysteine linkers, thioether linkers and click chemistry linkers.

10. The inhibitor of miR-574-5p for use according to at least one of the preceding claims, wherein the inhibitor is an antagomiR comprising any of SEQ ID NO: 2, 3, 4, 5, or 42.

11. The inhibitor of miR-574-5p for use according to at least one of the preceding claims, wherein the nucleic acid is optionally additionally conjugated to a protein which specifically binds to an osteoclast surface marker selected from the group consisting of tartrate-resistant acid phosphatase (TRAP) SEQ ID NO: 34, cathepsin K (cath K) SEQ ID NO: 35, calcitonin receptor (CTR) SEQ ID NO: 36, matrix metalloproteinase 9 (MMP9) SEQ ID NO: 37, and carbonic anhydrase II (CAII) SEQ ID NO: 38, as well as combinations thereof.

12. The inhibitor of miR-574-5p for use according to at least one of the preceding claims, wherein the inhibitor is embedded in and/or conjugated to an arthritogenic virus vector, optionally an alphavirus selected from the group consisting of chikungunya virus (CHIKV), o'nyong-nyong virus (ONNV), Ross River virus (RRV), Barmah Forest virus (BFV), Mayaro virus (MAYV), Sindbis virus (SINV) and Semliki Forest virus (SFV), as well as combinations thereof.

13. The inhibitor of miR-574-5p for use according to at least one of the preceding claims, wherein the inhibitor is embedded in and/or conjugated to an intra-articular hyaluronic acid (IAHA).

14. Pharmaceutical composition comprising the inhibitor according to at least one of the preceding claims.

15. Pharmaceutical composition according to claim 14, wherein the composition further comprises a carrier material selected from the group consisting of intra-articular hyaluronic acid (IAHA), liposomes, nanocarriers (NCs) and/or cholesterol.
